(19) Europäisches Patentamt
European Patent Office
Office européen des brevets

(11) EP 1 726 295 B1

(12) **FASCICULE DE BREVET EUROPEEN**

(45) Date de publication et mention de la délivrance du brevet:
**09.06.2010 Bulletin 2010/23**

(21) Numéro de dépôt: **06113858.2**

(22) Date de dépôt: **12.05.2006**

(51) Int Cl.:
***A61K 8/36*** *(2006.01)* ***A61K 8/44*** *(2006.01)*
***A61K 8/45*** *(2006.01)* ***A61K 8/46*** *(2006.01)*
***A61K 8/898*** *(2006.01)* ***A61K 8/73*** *(2006.01)*
***A61Q 5/02*** *(2006.01)* ***A61Q 5/12*** *(2006.01)*
***A61Q 1/14*** *(2006.01)*

(54) **Compositions cosmétiques détergentes comprenant une silicone aminée pipéridine et trois tensioactifs et utilisation de ces dernières**

Kosmetische Waschmittelzusammensetzungen enthaltend ein Amino Piperidin Silikon und drei Tenside und ihre Verwendung

Cosmetic detergent compositions comprising an amino silicone with a piperidine group and three surfactants and use thereof

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **24.05.2005 FR 0551350**

(43) Date de publication de la demande:
**29.11.2006 Bulletin 2006/48**

(73) Titulaire: **L'Oréal**
**75008 Paris (FR)**

(72) Inventeur: **Maubru, Mireille**
**78400 Chatou (FR)**

(74) Mandataire: **Le Blainvaux Bellegarde, Françoise**
**L'Oréal**
**D.I.P.I.**
**25-29 Quai Aulagnier**
**92600 Asnières (FR)**

(56) Documents cités:
**EP-A- 0 739 625** **EP-A- 0 974 335**
**WO-A-97/46211** **US-A1- 2003 134 760**

Remarques:
Le dossier contient des informations techniques présentées postérieurement au dépôt de la demande et ne figurant pas dans le présent fascicule.

EP 1 726 295 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention concerne une composition cosmétique conditionnante et détergente pour le soin et le lavage simultanés des matières kératiniques.
L'invention concerne aussi l'utilisation de ladite composition dans l'application susmentionnée.

Pour le nettoyage et/ou le lavage des cheveux et/ou de la peau, l'utilisation de compositions détergentes (shampooing ou gel-douche) à base essentiellement d'agents tensioactifs classiques de type notamment anionique, non ionique et/ou amphotère, mais plus particulièrement de type anionique, est courante. Ces compositions sont appliquées sur cheveux ou peau mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur les matières kératiniques des dommages plus ou moins marqués liés en particulier à l'élimination progressive des lipides ou protéines contenues dans ou à la surface de ces dernières.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer
ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes.

Toutefois, et malgré les progrès réalisés récemment dans le domaine des shampooings à base de polymères cationiques en particulier les dérivés cationiques de cellulose ou de gomme de guar et de silicone, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-avant, de meilleures performances.

On a déjà utilisé des silicones cationiques dans des compositions pour le lavage ou le soin des cheveux pour faciliter le démêlage des cheveux et pour leur communiquer douceur et souplesse par exemple dans la demande de brevet GB-A-1513672. Cependant, l'usage de ces silicones dans ce but présente divers inconvénients. En raison de leur forte affinité pour les cheveux, ces polymères se déposent de façon importante lors d'utilisations répétées, et conduisent à des effets indésirables tel qu'un toucher désagréable. Ces inconvénients sont accentués dans le cas de cheveux fins, qui manquent de tenue, de nervosité et de volume.

US2003/130760 décrit des compositions de shampooings comprenant une silicone à amine encombrée. EP 0 739 625 divulgue des compositions détergentes comprenant un tensioactif anionique, un tensioactif amphotère et un céramide. EP 0 974 335 et WO97/46211 divulguent des compositions détergentes comprenant un tensioactif anionique, un tensioactif amphotère et une silicone aminée.

Le but de la présente invention est d'obtenir des compositions détergentes suffisamment moussantes ayant de bonnes propriétés cosmétiques et ne dégradant pas ou peu la couleur (coloration) synthétique (artificielle) des cheveux. De plus la couleur des compositions ne doit pas ou peu évoluer dans le temps.

Ainsi, après de nombreuses recherches menées sur la question, la Demanderesse a maintenant découvert, de façon totalement inattendue et surprenante, qu'en associant une silicone aminée particulière avec une association de tensioactif anionique de type carboxylique, de tensioactif anionique sulfate et de tensioactif amphotère, il est possible d'obtenir des compositions détergentes présentant d'excellentes propriétés cosmétiques, en particulier de lissage, de démêlage, de douceur et de brillance et de volume des matières kératiniques traitées et ceci tout en conservant leur bon pouvoir lavant intrinsèque, leur pouvoir moussant et leurs autres propriétés cosmétiques.

Les compositions conformes à l'invention confèrent aux matières kératiniques notamment les cheveux, un remarquable effet traitant qui se manifeste notamment par une facilité de démêlage, ainsi qu'un apport de volume, de légèreté, de lissage, de douceur et de souplesse sans aucun effet de charge. Les cheveux ont un aspect naturel, propre et non gras.
Par ailleurs, la couleur des compositions selon l'invention n'évolue pratiquement pas au cours du temps. Enfin, les compositions selon l'invention ont un effet peu dégradant vis-à-vis de la coloration synthétique (artificielle) des cheveux. Les propriétés moussantes sont très bonnes, la mousse est abondante et se rince facilement.

La présente invention a ainsi pour objet une nouvelle composition cosmétique conditionnante et détergente, caractérisée par le fait qu'elle comprend, dans un milieu aqueux :

(A) au moins un agent tensioactif anionique de type sulfate et/ou sulfonate,
(B) au moins un agent tensioactif anionique de type carboxylique différent de l'agent tensioactif (A)
(C) au moins un agent tensioactif amphotère,
(D) au moins une silicone aminée comprenant une fonction amine portée par un groupe stériquement encombré, telle que définie ci-après,

le rapport en poids tensioactif anionique sulfate ou sulfonate / tensioactif anionique carboxylique allant de 2 à 25.

La présente invention a également pour objet l'utilisation de la composition selon l'invention pour le soin et le lavage simultanés des matières kératiniques telles que les cheveux et la peau.

Une description détaillée de la présente invention va maintenant être donnée.

(A) Tensioactifs sulfate(s) ou sulfonate(s)

Selon l'invention, les tensioactifs anioniques sulfate(s) ou sulfonate(s) sont des tensioactifs anioniques comportant au moins une fonction sulfate ($-OSO_3H$ ou $-OSO_3^-$) et/ou une fonction sulfonate ($-SO_3H$ ou $-SO_3^-$).

Les tensioactifs anioniques sulfates ou sulfonates utilisables, seuls ou mélanges, dans le cadre de la présente invention, sont les sels (en particulier sels alcalins, notamment de sodium, sels d'ammonium, sels d'amines, sels d'aminoalcools ou sels de magnésium) des alkylsulfates, alkylamidosulfates, alkyléthersulfates, alkylamidoéthersulfates, alkylaryléthersulfates, des alkyléthersulfosuccinates, des acyl iséthionates, des méthyl acyl taurates; le radical alkyle ou acyle de tous ces différents composés comportant de préférence de 8 à 24 atomes de carbone, et le radical aryle désignant de préférence un groupement phényle ou benzyle.

Le nombre moyen de groupements oxyde d'éthylène ou oxyde de propylène peut aller notamment de 2 à 50 et plus particulièrement de 2 à 10.

Parmi ces tensioactifs anioniques, on préfère utiliser les sels d'alkyléthersulfates en $C_8$-$C_{14}$ et plus particulièrement ceux en $C_{12}$-$C_{14}$. Ces sels comprennent de préférence de 2 à 5 groupements d'oxyde d'éthylène. On utilise de préférence un agent tensioactif anionique choisi parmi les alkyl($C_{12}$-$C_{14}$) sulfates de sodium, de triéthanolamine, de magnésium ou d'ammonium, les alkyl ($C_{12}$-$C_{14}$)éthersulfates de sodium, d'ammonium ou de magnésium oxyéthylénés à 2,2 moles d'oxyde d'éthylène, le cocoyl iséthionate de sodium et les méthyl acyl(C8-C20)taurates.

Les tensioactifs anioniques sulfates ou sulfonates sont généralement présents à raison de 2,5 % à 25 % en poids, de préférence de 5 à 25 % en poids, plus particulièrement de 8 à 20% en poids, mieux de 9 à 15% en poids par rapport au poids total de la composition.

(B) Tensioactifs anioniques carboxyliques

Selon l'invention, les tensioactifs anioniques carboxyliques sont des tensioactifs anioniques comportant au moins une fonction carboxylique (-COOH) éventuellement sous forme de sel (-COO-).

Les tensioactifs anioniques de type carboxyliques différents des tensioactifs (A) ne comprennent de préférence pas de fonction sulfate ou sulfonate et peuvent être notamment choisis parmi les acides d'alkyl D galactoside uroniques et leurs sels, les acides alkyl ($C_6$-$C_{24}$) éther carboxyliques polyoxyalkylénés, les acides alkyl($C_6$-$C_{24}$)aryl éther carboxyliques polyoxyalkylénés et leurs sels, les acides alkyl($C_6$-$C_{24}$) amido éther carboxyliques polyoxyalkylénés et leurs sels, en particulier ceux comportant de 2 à 50 groupements oxyde d'alkylène en particulier d'éthylène tels que les composés proposés par la société KAO sous les dénominations AKYPO, les acyl($C_6$-$C_{24}$) sarcosinates et leurs sels, les acyl ($C_6$-$C_{24}$)lactylates et leurs sels et les acyl($C_6$-$C_{24}$) glutamates. On peut également utiliser les esters d'alkyl($C_6$-$C_{24}$) polyglycosides carboxyliques tels que les alkylglucoside acétates, les alkylglucoside citrates et les alkylpolyglycoside tartrate. De tels produits sont notamment vendus sous les dénominations de EUCAROL APG/EC et EUCAROL APG/ET par la société LAMBERTI et PLANTAPON LGC SORB par la société COGNIS.

On peut également utiliser les mélanges de ces tensioactifs.

Les sels sont en particulier choisis parmi les sels alcalins, notamment de sodium, les sels d'ammonium, les sels d'amines, les sels d'aminoalcools tels la triéthanolamine ou la monoéthanolamine et les sels de magnésium.

Les tensioactifs anioniques du type acides ou sels d'éthers carboxyliques polyoxyalkylénés sont en particulier ceux qui répondent à la formule (1) suivante :

$$R_1-(OC_2H_4)_n-OCH_2COOA \qquad (1)$$

dans laquelle :

R1 représente un radical ou un mélange de radicaux alkyle ou alcényle linéaire ou ramifié en $C_8$-$C_{22}$, un radical alkyl($C_8$-$C_9$)phényle, un radical R2CONH-$CH_2$-$CH_2$- avec R2 désignant un radical alkyle ou alcényle linéaire ou ramifié en $C_9$-$C_{21}$,
et n est un nombre entier ou décimal (valeur moyenne) pouvant varier de 2 à 24 et de préférence de 2 à 10, le radical alkyle ayant entre 6 et 20 atomes de carbone environ, de préférence de 8 à 18 atomes de carbone et aryle désignant de préférence phényle,

A désigne H, ammonium, Na, K, Li, Mg ou un reste monoéthanolamine ou triéthanolamine. On peut également utiliser des mélanges de composés de formule (1) en particulier des mélanges dans lesquels les groupements $R_1$ sont différents.

Les acides éther carboxylique oxyalkylénés ou leurs sels utilisés de préférence selon la présente invention sont choisis parmi ceux de formule (I) dans laquelle R1 désigne un radical ou un mélange de radicaux alkyl($C_{12}$-$C_{14}$), cocoyle, oléyle; un radical nonylphényle ou octylphényle, A désigne un atome d'hydrogène ou de sodium, et n varie de 2 à 20 et de préférence 2 à 10.
On utilise de préférence les acides alkyl ($C_6$-$C_{24}$) éther carboxyliques polyoxyalkylénés et leurs sels, les acides alkyl ($C_6$-$C_{24}$) amido éther carboxyliques polyoxyalkylénés en particulier ceux comportant de 2 à 15 groupements oxyde d'alkylène, et leurs sels et leurs mélanges.

Plus préférentiellement encore, on utilise des composés de formule (I) dans laquelle R désigne un radical alkyl ($C_{12}$), A désigne un atome d'hydrogène ou de sodium, et n varie de 2 à 10.

Parmi les produits commerciaux, on peut utiliser de préférence les produits vendus par la société CHEM Y sous les dénominations :

AKYPO® NP 70 (R=nonylphényle, n=7, p=0, A=H)
AKYPO® NP 40 (R=nonylphényle, n=4, p=0, A=H)
AKYPO® OP 40 (R=octylphényle, n=4, p=0, A=H)
AKYPO® OP 80 (R=octylphényle, n=8, p=0, A=H)
AKYPO® OP 190 (R=octylphényle, n=19, p=0, A=H)
AKYPO® RLM 38 (R= alkyl($C_{12}$-$C_{14}$), n=3,8, p=0, A=H)
AKYPO® RLM 38 NV (R= alkyl($C_{12}$-$C_{14}$), n=4, p=0, A=Na)
AKYPO® RLM 45 (R= alkyl($C_{12}$-$C_{14}$), =4,5, p=0, A=H)
AKYPO® RLM 45 NV (R= alkyl($C_{12}$-$C_{14}$), n=4,5, p=0, A=Na)
AKYPO® RLM 100 (R= alkyl($C_{12}$-$C_{14}$), n=10, p=0, A=H)
AKYPO® RLM 100 NV (R= alkyl($C_{12}$-$C_{14}$), n=10, p=0, A=Na)
AKYPO® RLM 130 (R= alkyl($C_{12}$-$C_{14}$), n=13, p=0, A=H)
AKYPO® RLM 160 NV (R= alkyl($C_{12}$-$C_{14}$), n=16, p=0, A=Na)
ou par la société SANDOZ sous les dénominations :
SANDOPAN DTC-Acid (R= alkyl($C_{13}$), n=6, p=0, A=H)
SANDOPAN DTC (R= alkyl($C_{13}$), n=6, p=0, A=Na)
SANDOPAN LS 24 (R= alkyl($C_{12}$-$C_{14}$), n=12, p=0, A=Na)
SANDOPAN JA 36 (R= alkyl($C_{13}$), n=18, p=0, A=H),

et plus particulièrement, les produits vendus sous les dénominations suivantes :

AKYPO® RLM 45

AKYPO® RLM 100

AKYPO® RLM 38.

On utilise de préférence les acides alkyl ($C_6$-$C_{24}$) éther carboxyliques polyoxyalkylénés et leurs sels, les acides alkyl($C_6$-$C_{24}$) amido éther carboxyliques polyoxyalkylénés et leurs sels en particulier ceux comportant de 2 à 15 groupements oxyde d'alkylène et les esters d'alkyl($C_6$-$C_{24}$)polyglycosides carboxyliques et leurs sels et leurs mélanges.

Les tensioactifs anioniques carboxyliques différents des tensioactifs cités en A) sont généralement présents à raison de 0,1 à 15%, de préférence de 0,5 à 10 % en poids, plus particulièrement de 1 à 5% en poids et encore plus préférentiellement de 1,5 à 3% en poids par rapport au poids total de la composition.

(C) Tensioactif(s) amphotère(s) et/ou zwittérioniques:

Les agents tensioactifs amphotères et/ou zwittérioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

Parmi les dérivés d'amines, on peut citer les produits décrits dans les brevets US-2 528 378 et US-2 781 354 et de structures :

$$R_2\text{-CONHCH}_2\text{CH}_2\text{-N}(R_3)(R_4)(\text{CH}_2\text{COO-}) \qquad (2)$$

dans laquelle : $R_2$ CO désigne un radical acyle en C6-C24, par exemple un radical présent dans l'huile de coprah hydrolysée, un radical octoyle, décoyle ou dodécanoyle et leurs mélanges, $R_3$ désigne un groupement bêta-hydroxyéthyle et $R_4$ un groupement carboxyméthyle ; et

$$R_2\text{-CONHCH}_2\text{CH}_2\text{-N(B)(C)} \qquad (3)$$

dans laquelle :

B représente $-CH_2CH_2OX'$, C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$ - CHOH - $SO_3H$
$R'_2$ CO désigne un radical acyle en C6-C24, par exemple un radical présent dans l'huile de coprah hydrolysée ou l'huile de lin, un radical octoyle, décoyle ou dodécanoyle, stéaroyle ou isostéaroyle, oléoyle et leurs mélanges.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHODIA CHIMIE.

Selon la présente invention, on préfère plus particulièrement utiliser les agents tensio-actifs amphotères appartenant au groupe des bétaïnes tels que les alkylbétaïnes en particulier la cocoylbétaïne commercialisée sous la dénomination « DEHYTON AB 30 » en solution aqueuse à 30 % de MA par la société HENKEL ou les alkylamidobétaines, en particulier la cocamidopropylbétaine telles que la TEGOBETAINE® F50 commercialisée par la société GOLDSCHMIDT.

Le ou les tensio-actif(s) amphotère(s) sont généralement présents dans des quantités allant de 1 à 20 % en poids, de préférence de 1,5 à 15 % en poids, et plus particulièrement de 1,5 à 10% en poids, mieux de 3 à 7% en poids par rapport au poids total de la composition.

La quantité minimale de tensioactifs est celle suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la quantité totale de tensioactifs peut représenter de 4 % à 50 % en poids, de préférence de 6 % à 35 % en poids, et encore plus préférentiellement de 8 % à 25 % en poids, mieux de 12 à 20% en poids du poids total de la composition finale.

Le rapport en poids tensioactif(s) anionique(s) sulfate(s) ou sulfonate(s) / tensioactif(s) amphotère(s) va de préférence de 0,5 à 12, plus particulièrement de 1 à 10, de 1,5 à 7 et encore plus préférentiellement de 1,5 à 5.

Le rapport en poids tensioactif(s) anionique(s) sulfate(s) ou sulfonate(s) / tensioactif(s) anionique(s) carboxylique(s) va de préférence de 1 à 30 et encore plus préférentiellement de 2 à 25.

Le rapport en poids tensioactif(s) anionique(s) carboxylique(s)/ tensioactif(s) amphotère(s) va généralement de 0,3 à 20, de préférence de 0,5 à 15 et plus préférentiellement de 1 à 10.

Le rapport en poids tensioactifs de type carboxylate/totalité des tensioactifs peut varier de 0,01 à 0,5 et de préférence de 0,02 à 0,3.

D) Silicone aminée stériquement encombrée

Par silicone aminée comportant une fonction amine portée par un groupement stériquement encombré, on entend au sens de la présente invention toute silicone possédant à l'une au moins de ses extrémités et/ou sur des chaînes pendantes au moins un groupement aminé intégré dans un hétérocycle composé de 5 à 8 chaînons, les carbone en $\alpha$, et $\alpha'$ de l'atome d'azote étant totalement substitués par des atomes ou groupements différents de l'hydrogène.

Les silicones aminées imposées dans l'invention, comprenant une fonction amine portée par un groupe stériquement encombré sont des polyorganosiloxanes ayant par mole au moins un motif de formule générale (I) :

$$\text{(I)} \qquad (R)_a(X)_bZSi(O)_{\frac{3-(a+b)}{2}}$$

dans laquelle :

- les symboles R sont identiques ou différents et représentent un radical hydrocarboné monovalent choisi parmi les radicaux alkyles, linéaires ou ramifiés, ayant de 1 à 4 atomes de carbone, phényle, benzyle et trifluro-3,3,3 propyle;
- les symboles X sont identiques ou différents et représentent un radical monovalent choisi parmi un groupement hydroxyle et un radical alkoxy, linéaire ou ramifié, ayant de 1 à 3 atomes de carbone ;
- Z représente un radical monovalent comprenant une fonction amine portée par un groupe stériquement encombré, à savoir un ou plusieurs groupe(s) pipéridinyle(s) stériquement encombrés choisi parmi :

1) les radicaux de formule (II) :

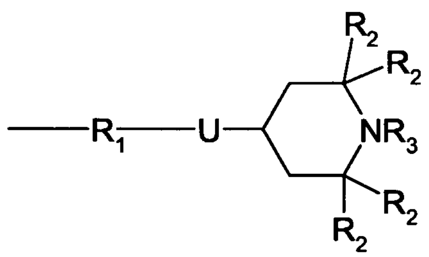

(II)

dans laquelle :

A) $R_1$ est un radical hydrocarboné divalent comprenant éventuellement un ou plusieurs hétéroatomes tels que O et N choisi parmi :

a) Les radicaux alkylènes, linéaires ou ramifiés, ayant de 2 à 18 atomes de carbone ;
b) Les radicaux alkylène-carbonyle dont la partie alkylène, linéaire ou ramifiée, comporte 2 à 20 atomes de carbone ;
c)Les radicaux alkylène-cyclohexylène dont la partie alkylène, linéaire ou ramifiée, comporte de 1 à 12 atomes de carbone et la partie cyclohexylène comporte un groupement OH et éventuellement 1 ou 2 radicaux alkyles ayant de 1 à 4 atomes de carbone ;
d)Les radicaux de formule —$R_4$-O-$R_5$— dans laquelle les radicaux R4 et R5 identiques ou différents représentent des radicaux alkylènes ayant 1 à 12 atomes de carbone;
e)Les radicaux de formule —$R_4$-O-$R_5$— dans laquelle les radicaux $R_4$ et $R_5$ ont les significations indiquées précédemment l'un d'entre eux ou les deux sont substitués par un ou deux groupement(s) OH ;
f) Les radicaux de formules -$R_4$-COO-$R_5$- et -$R_4$-OCO-$R_5$- dans lesquelles $R_4$ et $R_5$ ont les significations précédentes ;
g)Les radicaux de formule -$R_6$-O-$R_7$-O-CO-$R_8$- dans laquelle $R_6$, $R_7$ et $R_8$, identiques ou différents, représentent des radicaux alkylènes ayant de 2 à 12 atomes de carbone et le radical $R_7$ est éventuellement substitué par un groupement hydroxyle ;

B) U représente -O- ou -$NR_9$-, $R_9$ étant un radical monovalent choisi parmi un atome d'hydrogène un radical

alkyle, linéaire ou ramifié, ayant de 1 à 6 atomes de carbone; un radical divalent -$R_1$- ayant la signification indiquée précédemment, l'un des liens valentiels étant relié à l'atome d'azote de -$NR_9$-, l'autre étant relié à un atome de silicium ;

C)$R_2$ sont des radicaux, identiques ou différents, choisis parmi les radicaux alkyles, linéaires ou ramifiés, ayant de 1 à 3 atomes de carbone et phényle ;

D)$R_3$ représente un atome d'hydrogène ou le radical $R_2$ ;

- a est un nombre choisi parmi 0, 1 et 2 ;
- b est un nombre choisi parmi 0, 1, et 2 ;
- la somme a + b est au plus égale à 2.

Le polyorganosiloxane utilisé peut comprendre en outre au moins un autre motif siloxyle de formule :

$$\text{(III)} \qquad (R)_c(X)_dVSi(O)_{\frac{3-(c+d)}{2}}$$

dans laquelle :

- les symboles R et X ont les mêmes significations que celles données ci avant à propos de la formule (I) ;
- le symbole V représente: un radical alkyle, linéaire ou ramifié, ayant de 5 à 20 atomes de carbone; un radical de formule -$(CH_2)$p-$COOR_{12}$ dans laquelle p représente un nombre de 5 à 20 et $R_{12}$ représente un radical alkyle linéaire ou ramifié de 1 à 12 atomes de carbone ; un radical de formule -$(CH_2)_q$-O-$R_{13}$ dans laquelle q représente un nombre de 3 à 10 et $R_{13}$ représente un atome d'hydrogène, un oxyde d'éthylène, un groupement mono ou polyoxyéthyléné, un groupement mono ou polyoxypropyléné, un enchaînement mixte constitué de motifs d'oxyde d'éthylène et d'oxyde de propylène ou un radical acyle ayant de 2 à 12 atomes de carbone ;
- c est un nombre choisi parmi 0, 1 et 2 ;
- d est un nombre choisi parmi 0, 1 et 2 ;
- la somme c + d est au plus égale à 2.

Le polyorganosiloxane mis en oeuvre peut comprendre en outre d'autre(s) motif(s) siloxyle(s) de formule (IV):

$$\text{(IV)} \qquad (R)_e(X)_fSi(O)_{\frac{4-(e+f)}{2}}$$

dans laquelle :

- R et X ont les mêmes significations que celles données à propos de la formule (I) ;
- e est un nombre choisi parmi 0, 1, 2 et 3 ;
- f est un nombre choisi parmi 0, 1, 2 et 3 ;
- La somme e + f est au plus égale à 3.

Les motifs siloxyles de formule (I), quand il y en a plus de deux, peuvent être identiques ou différents entre eux; la même remarque s'applique également aux motifs siloxyles de formules (III) et (IV).

Les différents motifs sont de préférence répartis statistiquement le long de la chaîne.

Le polyorganosiloxane mis en oeuvre peut présenter une structure linéaire cyclique ou ramifiée (présence de motifs de types T et/ou Q) ou mélange de ces structures. Dans le cas de la présence de motifs monoorganosiloxy de type T et/ou de motifs Q ($SiO_2$), ces motifs sont dans la proportion d'au plus 10% par rapport au nombre de motifs diorganosiloxy de type D.

Le polyorganosiloxane mis en oeuvre est, par exemple, un polydiorganosiloxane linéaire de formule moyenne (V) :

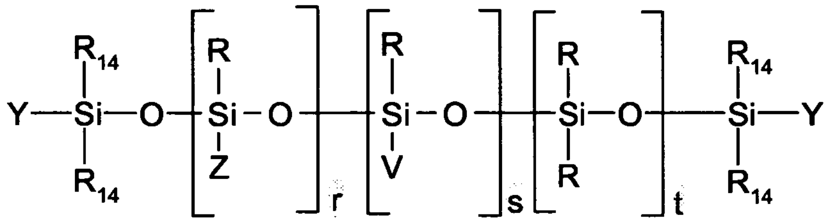

(V)

Dans laquelle :

- Les différents motifs sont de préférence répartis statistiquement le long de la chaîne.
- Les symboles R, Z et V ont les significations données ci avant à propos des formules (I) et (III) ;
- Le symbole Y représente un radical monovalent choisi parmi les radicaux R, Z, V et X;
- Les symboles $R_{14}$ sont identiques ou différents et représentent un radical monovalent choisi parmi un radical R et un radical X tel que défini ci avant à propos de la formule (I) ;
- r, s et t sont égaux à zéro ou représentent des nombres entiers ou fractionnaires supérieurs à zéro, avec la condition supplémentaire selon laquelle si r = 0, au moins un des deux radicaux Y représente le radical Z.

De préférence, la composition selon l'invention comprend un polyorganosiloxane à motifs(s) de formules (I) + éventuellement (III) + éventuellement (IV) ou un polyorganosiloxane de formule (V) :

Ces polyorganosiloxanes ont en moyenne par mole : de 2 à 1600 atomes de silicium, de 1 à 100 radicaux Z tels que ceux ci-après définis et de 0 à 50 radicaux V tels que ceux ci-après définis;

- et dans la structure duquel :
  - R est choisi parmi les radicaux méthyle, éthyle, n-propyle et isopropyle, de préférence méthyle ;
  - X est choisi parmi les radicaux hydroxyle, méthoxy et éthoxy ;
  - Z est choisi parmi les radicaux à groupes(s) pipéridinyle(s) :

1) de formule (II) dans laquelle :

A) $R_1$ est un radical hydrocarboné divalent comprenant éventuellement un ou plusieurs hétéroatomes tels que O et N choisi parmi :

a) Les radicaux alkylènes, linéaires ou ramifiés, ayant de 2 à 18 atomes de carbone ;
b) Les radicaux alkylènes, linéaires ou ramifiés, ayant de 3 à 12 atomes de carbone ;
c) Le radical $-(CH_2)_{10}-CO-$;
d) Les radicaux alkynèle-cyclohexylène dont la partie alkylène, linéaire ou ramifiée, comporte de 2 à 6 atomes de carbone et la partie cyclohylène comporte un groupement -0H et éventuellement 1 ou 2 subsistants alkyles ayant 1 à 4 atomes de carbone ;
e) Les radicaux de formule $-R_4-O-R_5-$ dans laquelle les radicaux $R_4$ et $R_5$ identiques ou différents représentent des radicaux alkylènes ayant 2 à 6 atomes de carbone ;
f) Les radicaux de formule $-R_4-O-R_5-$ dans laquelle les radicaux $R_4$ et $R_5$ ont les significations indiquées précédemment et R5 est substitué par un groupe OH ;
g) Les radicaux de formules $-R_4-COO-R_5-$ et $-R_4-OCO-R_5-$ dans lesquelles $R_4$ et $R_5$ ont les significations précédentes ;
h) Les radicaux de formule $-R_6-O-R_7-O-CO-R_8-$ dans laquelle $R_6$, $R_7$, $R_8$, identiques ou différents, représentent des radicaux alkylènes ayant de 2 à 6 atomes de carbone et le radical $R_7$ est substitué par un groupement hydroxyle ;

B) U représente -O- ou -$NR_9$- où $R_9$ est un radical alkyle, linéaire ou ramifié, ayant de 1 à 4 atomes de carbone;
C) $R_2$ représente un radical méthyle;

- V est choisi parmi: un radical alkyle, linéaire ou ramifié, ayant 5 à 18 atomes de carbone; un radical de formule $-(CH_2)_{10}-COO-R_{12}$ laquelle $R_{12}$ représente un radical alkyle, linéaire de 1 à 6 atomes de carbone, un radical de formule $-(CH_2)_3-O-R_{13}$, où $R_{13}$ représente un atome d'hydrogène, un oxyde d'éthylène, un groupement mono ou polyoxyéthyléné, un groupement mono ou polyoxypropyléné, un enchaînement mixte constitué de motifs d'oxyde d'éthylène et d'oxyde de propylène ou un groupement acyle ayant de 2 à 6 atomes de carbone.

Dans le cadre de cette modalité préférentielle, le polydiorganosiloxane linéaire de formule (V) présente les valeurs suivantes pour les symboles r, s et t :

- r est un nombre entier ou fractionnaire allant de 0 à 98 avec la condition selon laquelle si r = 0, au moins un des deux radicaux Y représente le radical Z
- s est un nombre entier ou fractionnaire allant de 0 à 48 ;
- t est un nombre entier ou fractionnaire allant de 0 à 1598 ;
- la somme r + s + t est un nombre entier ou fractionnaire allant de 0 à 1598.

De manière plus préférentielle, la composition selon l'invention comprend un polyorganosiloxane à motifs de formule (I) + (IV) + éventuellement (III) ou un polyorganosiloxane de formule (V) :

- qui a en moyenne par mole : de 5 à 800 atomes de silicium, de 1 à 60 radicaux Z tels que ceux ci-après définis et de 0 à 20 radicaux V tels que ceux ci-après définis ;
- et dans la structure duquel :
  - R est un radical méthyle ;
  - X est choisi parmi les radicaux hydroxyle et méthyle ;
  - Z est choisi parmi les radicaux à groupe(s) pipéridinyle(s) :
    - ➢ De formule (II) dans laquelle :
      - $R_1$ représente un radical: triméthylène ; décaméthylène-carbonyle ; hydroxy-2 oxa-4 heptaméthylène ; hydroxy-6 dioxa-4,8 oxo-3 undécaméthylène, de préférence triméthylène ;
        U représente -O- ou $NR_9$ où $R_9$ est choisi parmi les radicaux méthyle, éthyle, n-propyle et n-butyle ;
      - $R_2$ représente un radical méthyle;
      - $R_3$ représente un atome d'hydrogène;
  - V est choisi parmi les radicaux n-octyle et décaméthylène carboxylate de méthyle ou d'éthyle
    Dans le cadre de cette modalité plus préférentielle, le polydiorganosiloxane linéaire de formule (V) présente les valeurs suivantes pour les symboles r, s et t :
  - r est un nombre entier ou fractionnaire allant de 0 à 58 avec la condition selon laquelle si r = 0, au moins un des deux radicaux Y représente le radical Z ;
  - s est un nombre entier ou fractionnaire allant de 0 à 18 ;
  - t est un nombre entier ou fractionnaire allant de 3 à 798 ;
  - La somme r + s + t est un nombre entier ou fractionnaire allant de 3 à 798.

Généralement, 100 g de polyorganosiloxane tel que ceux précédemment définis comprennent 1 à 200 milliéquivalents (meq) de groupe(s) pipéridinyle(s) encombrés(s), de préférence de 2 à 100 et plus particulièrement de 5 à 50 meq.
Le pourcentage d'azote de la silicone aminée va de préférence de 0,05% à 1% en poids par rapport au poids total de la silicone, de préférence de 0,1 à 0,5% en poids.

Les silicones préférées selon l'invention présentent notamment la structure suivante :

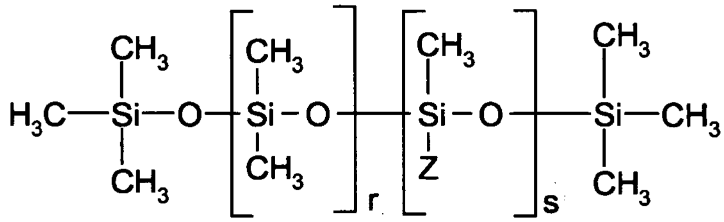

avec Z =

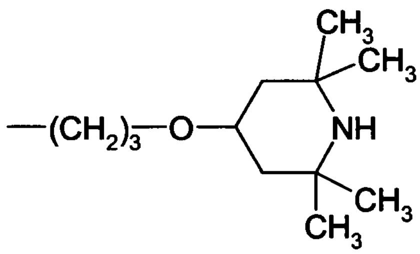

s varie de 1 à 50, de préférence de 2 à 30
r varie de 50 à 2000, de préférence de 500 à 1700.

De telles silicones aminées comprenant un groupe fonctionnel stériquement encombré sont par exemple proposées par la société RHODIA sous la dénomination RHODORSIL® Huile 21645, RHODORSIL® Huile 21650, JLR 3442 et JLR 3440.

Les silicones aminées à groupements stériquement encombrés peuvent se présenter sous forme de solutions, de dispersions, de microémulsions ou d'émulsions.

Selon l'invention, la ou les silicones aminées selon l'invention peuvent représenter de 0,001 % à 10 % en poids, de préférence de 0,01 % à 5% en poids et plus particulièrement de 0,1 à 3% en poids par rapport au poids total de la composition finale.

Les compositions de l'invention peuvent contenir en outre avantageusement au moins un autre agent tensioactif choisis parmi les tensioactifs non-ioniques, les tensioactifs cationiques ou leurs mélanges. On utilise de préférence un ou plusieurs tensioactifs non ioniques.

Les tensioactifs additionnels convenant à la mise en oeuvre de la présente invention sont notamment les suivants :

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools gras polyéthoxylés, polypropoxylés ou polyglycérolés, les alpha-diols gras polyéthoxylés, polypropoxylés ou polyglycérolés, les alkylphénols polyéthoxylés, polypropoxylés ou polyglycérolés ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensioactifs non-ioniques rentrant particulièrement bien dans le cadre de la présente invention. Les alcools gras polyoxyéthylénés et/ou

polyoxypropylénés sont également particulièrement utilisés selon la présente invention.

Les tensioactifs non ioniques peuvent représenter de 0,1 à 10% en poids, de préférence de 0,25 à 5% en poids par rapport au poids total de la composition.

Ainsi, selon l'invention, la quantité totale de tensioactifs peut représenter de 4 % à 50 % en poids, de préférence de 6 % à 35 % en poids, et encore plus préférentiellement de 8 % à 25 % en poids, mieux de 12 à 20% en poids du poids total de la composition finale.

Le milieu aqueux cosmétiquement acceptable peut être constitué uniquement par de l'eau ou par un mélange d'eau et d'un solvant cosmétiquement acceptable. tel qu'un alcool inférieur en $C_1$-$C_4$, comme l'éthanol, l'isopropanol, le tertiobutanol, le n-butanol ; les alkylèneglycols comme le propylèneglycol, l'hexylèneglycol ou le glycérol.

La composition selon l'invention comprend de préférence au moins 30% en poids d'eau et plus particulièrement de 50 à 90% en poids et encore préférentiellement de 70 à 85% en poids par rapport au poids total de la composition.

Les compositions détergentes selon l'invention présentent un pH final généralement compris entre 3 et 10. De préférence, ce pH est compris entre 5 et 8. L'ajustement du pH à la valeur désirée peut se faire classiquement par ajout d'une base (organique ou minérale) dans la composition, par exemple de la soude, de l'ammoniaque ou une (poly) amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination « AMINOL A15 » par la société CHEM Y, les acides polyacryliques réticulés et les copolymères acide acrylique / acrylates d'alkyle en $C_{10}$-$C_{30}$ réticulés. Ces agents régulateurs de viscosité sont utilisés dans les compositions selon l'invention dans des proportions pouvant aller jusqu'à 10 % en poids par rapport au poids total de la composition.

Les compositions conformes à l'invention peuvent également contenir jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les alcools gras, les éthers à chaînes grasses tels que par exemple le distéaryléther ou le 1-(hexadécyloxy)-2-octadécanol, les oxyde de titane.

Les compositions conformes à l'invention peuvent éventuellement contenir en outre d'autres agents ayant pour effet d'améliorer les propriétés cosmétiques de cheveux ou de la peau sans cependant altérer la stabilité des compositions. On peut citer à ce sujet les polymères anioniques ou non ioniques ou cationiques ou amphotères, les protéines, les hydrolysats de protéines, les céramides, les pseudocéramides, les huiles végétales, les acides gras notamment à chaînes linéaires ou ramifiées en $C_{16}$-$C_{40}$ tels que l'acide méthyl-18 eicosanoïque, les hydroxyacides, les vitamines, les provitamines telle que le panthénol, les silicones, aminées ou non aminées, volatiles ou non volatiles, solubles et insolubles dans le milieu différentes des silicones aminées selon l'invention, les filtres UV, les agents hydratants, les agents antipelliculaires ou antiséborrhéiques, les agents antichute des cheveux, les agents anti-radicaux libres, et leurs mélanges.

Selon un mode particulièrement préféré, les compositions selon l'invention comprennent en outre au moins un polymère cationique.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Les polymères cationiques utilisables selon l'invention ont de préférence une densité de charge cationique supérieure ou égale à 0,2 meq./g, et plus particulièrement comprise entre 0,2 et 8,5 meq./g.

Parmi tous les polymères cationiques susceptibles d'être utilisés dans le cadre de la présente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société MERCK, les polysaccharides cationiques non cellulosiques et plus particulièrement les gommes de guar modifiées par du chlorure de 2,3-époxypropyl triméthylammonium commercialisées par exemple sous la dénomination « JAGUAR C13S » par la société MEYHALL, les homopolymères et les copolymères éventuellement

réticulés de sel de (méth)acryloyloxyéthyltriméthylammonium, vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium), les copolymères quaternaires de vinylpyrrolidone et de sel de vinyl imidazole tels que les produits commercialisés par BASF sous les dénominations LUVIQUAT FC 370, LUVIQUAT FC 550, LUVIQUAT FC 905 et LUVIQUAT HM-552.

On peut également utiliser les polymères qui sont constitués de motifs récurrents répondant à la formule :

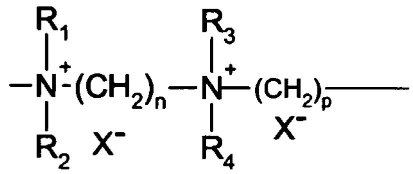
(I)

dans laquelle $R_1$, $R_2$, $R_3$ et $R_4$ identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.

Un composé de formule (I) particulièrement préféré est celui pour lequel $R_1$, $R_2$, $R_3$ et $R_4$, représentent un radical méthyle et n = 3, p = 6 et X = Cl, dénommé Hexadimethrine chloride selon la nomenclature INCI (CTFA).

Selon l'invention, le ou les polymères cationiques peuvent représenter de 0,001 % à 10 % en poids, de préférence de 0,005 % à 5 % en poids, et encore plus préférentiellement de 0,01 % à 3 % en poids, du poids total de la composition finale.

Les compositions selon l'invention peuvent contenir également des synergistes de mousses tels que des 1,2-alcanediols en $C_{10}$-$C_{18}$ ou des alcanolamides gras dérivés de mono ou de diéthanolamine ou d'isopropanolamine. On peut notamment citer le monoisopropanolamide d'acides de coprah.

Bien entendu, l'homme du métier veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.

Les modifications de la méthode sont les suivantes :

> La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et contenant 50 ml de la composition à tester. La mesure est faite 5 minutes après l'arrêt de l'écoulement de la composition.

Lorsque les compositions conformes à l'invention sont mises en oeuvre comme des shampooings, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Les compositions conformes à l'invention sont également utilisables comme gels douche, bains moussants, comme produits démaquillants moussants, pour le lavage et le conditionnement des cheveux et/ou de la peau, auquel cas ils sont appliqués sur la peau et/ou les cheveux humides et sont rincés après application.

Ces compositions peuvent se présenter sous la forme de liquides plus ou moins épaissis, de crèmes ou de gels et elles conviennent principalement au lavage, au soin des matières kératiniques en particulier des cheveux et de la peau et encore plus particulièrement des cheveux.

L'invention a également pour objet l'utilisation d'une composition telle définie ci-dessus pour le nettoyage et/ou le démaquillage des matières kératiniques.

L'invention a pour objet l'utilisation en cosmétique d'une composition telle définie ci-dessus pour le nettoyage et/ou le soin et/ou le conditionnement et/ou le coiffage des cheveux.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions selon l'invention sont utilisées de préférence comme shampooings pour le lavage et le conditionnement des cheveux et ils sont appliqués dans ce cas-là sur les cheveux humides dans des quantités efficaces

pour les laver, et la mousse générée par massage ou friction avec les mains est ensuite éliminée après un éventuel temps de pause, par rinçage avec de l'eau, l'opération pouvant être répétée une ou plusieurs fois.

Des exemples concrets, illustrant l'invention vont maintenant être donnés.

EXEMPLE 1 :

On a réalisé deux compositions de shampooings, l'une conforme à l'invention (composition A) et l'autre comparative (composition B) :(MA signifie Matière Active) :

| Composition | | Invention | Comparatif |
|---|---|---|---|
| Lauryléthersulfate de sodium (C12/C14 à 70/30 en poids) à 2.2 moles d'oxyde d'éthylène | | 11.2 g M.A. | 11.2 g M.A. |
| Cocoyl bétaïne à 30% de MA en solution aqueuse | | 5.1 g M.A. | 5.1 g M.A. |
| Acide lauryl éther carboxylique (4.5 OE) | | 3 g M.A. | 3 g M.A. |
| Hydroxyéthylcellulose réticulée à l'épichlorhydrine et quaternisée par la triméthylamine (JR400 de AMERCHOL) | | 0.7 g | 0.7 g |
| Propoxytetramethyl piperidinyl dimethicone C11-15 proposé sous la dénomination Jeesilc UAF par la société JEEN INTERNATIONAL | | 1.5 gMA | - |
| Polidiméthylsiloxane à groupements aminoéthyl iminobutyl commercialisé sous la dénomination DC2-8566 par la société DOW CORNING | | - | 1.5 gMA |
| Monoisopropanolamide d'acides de coprah | | 2 g | 2 g |
| Conservateurs, parfum | | q.s. | q.s. |
| Hydroxyde de sodium | q.s.p. | pH 5.3 | pH 5.3 |
| Eau déminéralisée | q.s.p. | 100 g | 100 g |

On effectue un shampooing en appliquant la composition A sur des chevelures de cheveux sensibilisés préalablement mouillés. On fait mousser le shampooing, puis on rince abondamment à l'eau.
On procède selon le même mode opératoire que ci-dessus avec la composition comparative B.

Un panel d'experts a évalué l'aspect des cheveux mouillés et séchés.

Résultats :

- sur cheveux mouillés, les racines plus décollées pour les cheveux traités avec la composition A selon l'inven-
- sur cheveux séchés, le lissage visuel et au toucher est amélioré pour les cheveux traités avec la composition A selon l'invention.

De plus, la coloration de la composition conforme à l'invention ne présente qu'une très faible évolution dans le temps, contrairement à la composition comparative dont le jaunissement est important.

**EXEMPLES 2 et 3:**

On a réalisé deux compositions de shampooings conformes à l'invention (MA signifie Matière Active) :

| Composition | Exemple 2 | Exemple 3 |
|---|---|---|
| - Lauryléthersulfate de sodium (C12/C14 - 70/30 en poids) à 2.2 moles d'oxyde d'éthylène | 11.2 g MA | 11.2 g MA |
| - Cocoyl bétaïne à 30% de MA | 5.1 g MA | 5.1 g MA |
| - Acide lauryl éther carboxylique (4.5 OE) (AKYPO RLM45 de KAO) | 0.5 g MA | 0.5 g MA |
| - Hydroxyéthylcellulose réticulée à l'épichlorhydrine et quaternisée par la triméthylamine, (JR400 de AMERCHOL) | 0.6 g | 0.6 g |
| - α,ω diméthylpoly diméthyl, méthyl (3-(2,2,6,6-tetraméthylpipéridin-4-yloxy)propyl) siloxane, (Rhodorsil huile 21645 de RHODIA) | 1 g | - |

(suite)

| Composition | Exemple 2 | Exemple 3 |
|---|---|---|
| - α,ω diméthylpoly diméthyl, méthyl (3-(2,2,6,6-tetraméthylpipéridin-4-yloxy)propyl) siloxane, (Rhodorsil huile 21650 de RHODIA) | - | 1 g |
| - Monoisopropanolamide d'acides de coprah | 0.5 g | 0.5 g |
| - Alcool cétylique oxyéthyléné (20 OE) oxypropyléné (5 OP) | 0.5 g | 0.5 g |
| Conservateurs, parfum | q.s. | q.s. |
| - Hydroxyde de sodium q.s.p. | pH 5.3 | pH 5.3 |
| - Eau déminéralisée q.s.p. | 100 g | 100 g |

Ces compositions apportent du lissage visuel et au toucher aux cheveux sensibilisés, qui ont également du volume et sont brillants.

## Revendications

1. Composition cosmétique conditionnante et détergente, **caractérisée par le fait qu'**elle comprend, dans un milieu aqueux :

(A) au moins un agent tensioactif anionique de type sulfate et/ou sulfonate,
(B) au moins un agent tensioactif anionique de type carboxylique différent de l'agent tensioactif (A)
(C) au moins un agent tensioactif amphotère,
(D) au moins une silicone aminée comprenant une fonction amine portée par un groupe stériquement encombré, à l'une au moins de ses extrémités et/ou sur des chaînes pendantes, ce groupe étant intégré dans un hétérocycle composé de 5 à 8 chaînons, les carbone en α, et α' de l'atome d'azote étant totalement substitués par des atomes ou groupements différents de l'hydrogène, est choisie parmi les polyorganosiloxanes ayant par mole au moins un motif de formule générale (I) :

$$\text{(I)} \qquad (R)_a(X)_bZSi(O)_{\frac{3-(a+b)}{2}}$$

dans laquelle :

- les symboles R sont identiques ou différents et représentent un radicalhydrocarboné monovalent choisi parmi les radicaux alkyles, linéaires ou ramifiés, ayant de 1 à 4 atomes de carbone, phényle, benzyle et trifluro-3,3,3 propyle;
- les symboles X sont identiques ou différents et représentent un radical monovalent choisi parmi un groupement hydroxyle et un radical alkoxy, linéaire ou ramifié, ayant de 1 à 3 atomes de carbone ;
- Z représente un radical monovalent comprenant une fonction amine portée par un groupe stériquement encombré à savoir un ou plusieurs groupe(s) pipéridinyle(s) stériquement encombrés choisi parmi :

3) les radicaux de formule (II) :

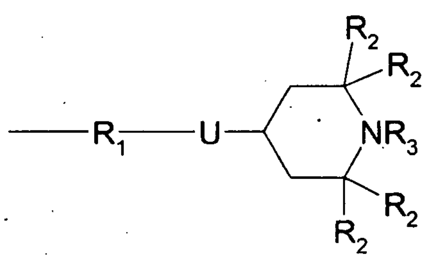

(II)

dans laquelle :

A) $R_1$ est un radical hydrocarboné divalent comprenant éventuellement un ou plusieurs hétéroatomes tels que O et N choisi parmi :

h) Les radicaux alkylènes, linéaires ou ramifiés, ayant de 2 à 18 atomes de carbone ;
i) Les radicaux alkylène-carbonyle dont la partie alkylène, linéaire ou ramifiée, comporte 2 à 20 atomes de carbone ;
j) Les radicaux alkylène-cyclohexylène dont la partie alkylène, linéaire ou ramifiée, comporte de 1 à 12 atomes de carbone et la partie cyclohexylène comporte un groupement OH et éventuellement 1 ou 2 radicaux alkyles ayant de 1 à 4 atomes de carbone ;
k)Les radicaux de formule $-R_4-O-R_5-$ dans laquelle les radicaux R4 et R5 identiques ou différents représentent des radicaux alkylènes ayant 1 à 12 atomes de carbone;
l) Les radicaux de formule $-R_4-O-R_5-$ dans laquelle les radicaux $R_4$ et $R_5$ ont les significations indiquées précédemment l'un d'entre eux ou les deux sont substitués par un ou deux groupement (s) OH ;
m) Les radicaux de formules $-R_4-COO-R_5-$ et $-R_4-OCO-R_5-$ dans lesquelles $R_4$ et $R_5$ ont les significations précédentes ;
n)Les radicaux de formule $-R_6-O-R_7-O-CO-R_8-$ dans laquelle $R_6$, $R_7$ et $R_8$, identiques ou différents, représentent des radicaux alkylènes ayant de 2 à 12 atomes de carbone et le radical $R_7$ est éventuellement substitué par un groupement hydroxyle ;

B) U représente -O- ou $-NR_9-$, $R_9$ étant un radical monovalent choisi parmi un atome d'hydrogène un radical alkyle, linéaire ou ramifié, ayant de 1 à 6 atomes de carbone; un radical divalent $-R_1-$ ayant la signification indiquée précédemment, l'un des liens valentiels étant relié à l'atome d'azote de $-NR_9-$, l'autre étant relié à un atome de silicium ;
C) $R_2$ sont des radicaux, identiques ou différents, choisis parmi les radicaux alkyles, linéaires ou ramifiés, ayant de 1 à 3 atomes de carbone et phényle ;
D) $R_3$ représente un atome d'hydrogène ou le radical $R_2$ ;

le rapport en poids tensioactif anionique sulfate ou sulfonate / tensioactif anionique carboxylique allant de 2 à 25.

**2.** Composition selon la revendication 1, **caractérisée en ce que** les tensioactifs anioniques sulfates et/ou sulfonates sont les sels des alkylsulfates, alkylamidosulfates, alkyléthersulfates, alkylamidoéthersulfates, alkylaryléthersulfates, des alkyléthersulfosuccinates, des acyl iséthionates, des méthyl acyl taurates; le radical alkyle ou acyle de tous ces différents composés comportant de préférence de 8 à 24 atomes dé carbone, et le radical aryle désignant de préférence un groupement phényle ou benzyle

**3.** Composition selon l'une quelconque des revendications 1 ou 2, **caractérisée en ce que** les tensioactifs anioniques de type carboxyliques différents des tensioactifs (A) sont choisis parmi les acides d'alkyl D galactoside uroniques et leurs sels, les acides alkyl ($C_6$-$C_{24}$), éther carboxyliques polyoxyalkylénés et leurs sels, les acides alkyl($C_6$-$C_{24}$) aryl éther carboxyliques polyoxyalkylénés et leurs sels, les acides alkyl($C_6$-$C_{24}$) amido éther carboxyliques polyoxyalkylénés et leurs sels, les acyl($C_6$-$C_{24}$) sarcosinates et leurs sels, les acyl($C_6$-$C_{24}$)lactylates et leurs sels et les acyl($C_6$-$C_{24}$) glutamates et leurs sels, les esters d'alkyl($C_6$-$C_{24}$)polyglycosides carboxyliques et leurs sels.

**4.** Composition selon l'une quelconque des revendications 1 à 3, **caractérisée en ce que** les agents tensioactifs amphotères et/ou zwittérioniques sont des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant ou les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.

**5.** Composition selon l'une quelconque des revendications 1 à 4, **caractérisée en ce que** le(s) tensioactif(s) anionique (s) sulfate et/ou sulfonate est (sont) présent(s) dans des concentrations allant de 2,5 à 25 % en poids, par rapport au poids total de la composition.

**6.** Composition selon l'une quelconque des revendications 1 à 5, **caractérisée en ce que** ledit(s) tensioactif(s) anionique(s) carboxylique(s) est(sont) présent(s) dans des concentrations allant de 0,1 à 15 % en poids, de préférence de 0,5 à 10 % en poids, par rapport au poids total de la composition.

7. Composition selon l'une quelconque des revendications 1 à 6, **caractérisée en ce que** le(s) tensioactif(s) amphotère (s) est(sont) présent(s) dans des concentrations allant de 0,1 à 20 % en poids, de préférence de 1 à 15 % en poids, par rapport au poids total de la composition.

8. Composition selon l'une quelconque des revendications 1 à 7, **caractérisée par le fait que** le rapport en poids tensioactif anionique sulfate ou sulfonate / tensioactif amphotère va de 0,5 à 12, plus particulièrement de 1 à 10 et encore plus préférentiellement de 1,5 à 5.

9. Composition selon l'une quelconque des revendications 1 à 8, **caractérisée par le fait que** le rapport en poids tensioactif anionique carboxylique/ tensioactif amphotère va de 0,3 à 20, de préférence de 0,5 à 15.

10. Composition selon l'une quelconque des revendications 1 à 9, **caractérisée par le fait que** la siliocne aminée comprend en outre au moins un autre motif siloxyle de formule (III) :

$$\text{(III)} \qquad (R)_c(X)_dVSi(O)_{\frac{3-(c+d)}{2}}$$

dans laquelle :

- les symboles R et X ont les mêmes significations que celles données ci avant à propos de la formule (I) ;
- le symbole V représente: un radical alkyle; linéaire ou ramifié, ayant de 5 à 20 atomes de carboné; un radical de formule $-(CH_2)_p-COOR_{12}$ dans laquelle p représente un nombre de 5 à 20 et - $R_{12}$ représente un radical alkyle linéaire ou ramifié de 1 à 12 atomes de carbone ; un radical de formule $-(CH_2)_q-O-R_{13}$ dans laquelle q représente un nombre de 3 à 10 et $R_{13}$ représente un atome d'hydrogène, un oxyde d'éthylène, un groupement mono ou polyoxyéthyléné, un groupement mono ou polyoxypropyléné, un enchaînement mixte constitué de motifs d'oxyde d'éthylène et d'oxyde de propylène ou un radical acyle ayant de 2 à 12 atomes de carbone ;
- c est un nombre choisi parmi 0, 1 et 2 ;
- d est un nombre choisi parmi 0, 1 et 2 ;
- la somme c + d est au plus égale à 2.

11. Composition selon l'une quelconque des revendications 1 à 10, **caractérisée par le fait que** la silicone aminée comprend en outre d'autre(s) motif(s) siloxyle(s) de formule (IV):

$$\text{(IV)} \qquad (R)_e(X)_fSi(O)_{\frac{4-(e+f)}{2}}$$

dans laquelle :

- R et X ont les mêmes significations que celles données à propos de la formule (I) ;
- e est un nombre choisi parmi 0, 1, 2 et 3 ;
- f est un nombre choisi parmi 0, 1, 2 et 3 ;
- La somme e + f est au plus égale à 3.

12. Composition selon l'une quelconque des revendications 1 à 11, **caractérisée par le fait que** la silicone aminée est un polyorganosiloxane linéaire de formule moyenne (V) :

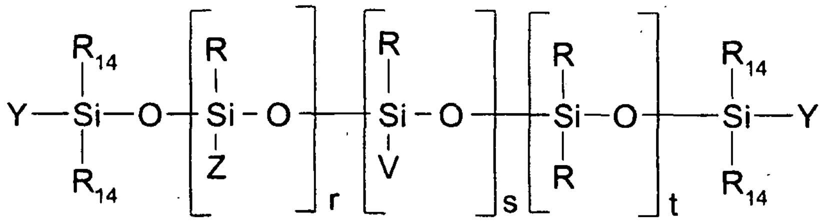

(V)

Dans laquelle :

• Les symboles R, Z et V ont les significations données ci avant à propos des formule (I) et (III) ;
• Le symbole Y représente un radical monovalent choisi parmi les radicaux R, Z, V et X;
• Les symboles $R_{14}$ sont identiques ou différents et représentent un radical monovalent choisi parmi un radical R et un radical X tel que défini ci avant à propos de la formule (I) ;
• r, s et t sont égaux à zéro ou représentent des nombres entiers ou fractionnaires supérieurs à zéro, avec la condition supplémentaire selon laquelle si r = 0, au moins un des deux radicaux Y représente le radical Z.

**13.** Composition selon l'une quelconque des revendications 1 à 12, **caractérisée par le fait que** la silicone aminée est un polyorganosiloxane à motifs(s) de formules (I) + éventuellement (III) + éventuellement (IV) ou un polyorganosiloxane de formule (V) :

ces polyorganosiloxanes ayant en moyenne par mole : de 2 à 1600 atomes de silicium, de 1 à 100 radicaux Z tels que ceux ci-après définis et de 0 à 50 radicaux V tels que ceux ci-après définis ;

- et dans la structure duquel :

• R est choisi parmi les radicaux méthyle, éthyle, n-propyle et isopropyle, de préférence méthyle ;
• X est choisi parmi les radicaux hydroxyle, méthoxy et éthoxy ;
• Z est choisi parmi les radicaux à groupes(s) pipéridinyle(s) :

2) de formule (II) dans laquelle :

A) $R_1$ est un radical hydrocarboné divalent comprenant éventuellement un ou plusieurs hétéroatomes tels que O et N choisi parmi :

i) Les radicaux alkylènes, linéaires ou ramifiés, ayant de 2 à 18 atomes de carbone ;
j) Les radicaux alkylènes, linéaires ou ramifiés, ayant de 3 à 12 atomes de carbone ;
k) Le radical $-(CH_2)_{10}-CO-$;
l) Les radicaux alkynèle-cyclohexylène dont la partie alkylène, linéaire ou ramifiée, comporte de 2 à 6 atomes de carbone et la partie cyclohylène comporte un groupement -0H et éventuellement 1 ou 2 subsistants alkyles ayant 1 à 4 atomes de carbone ;
m) Les radicaux de formule $-R_4-O-R_5-$ dans laquelle les radicaux $R_4$ et $R_5$ identiques ou différents représentent des radicaux alkylènes ayant 2 à 6 atomes de carbone ;
n) Les radicaux de formule $-R_4-O-R_5-$ dans laquelle les radicaux $R_4$ et $R_5$ ont les significations indiquées précédemment et $R_5$ est substitué par un groupe OH ;
o) Les radicaux de formules $-R_4-COO-R_5-$ et $-R_4-OCO-R_5-$ dans lesquelles $R_4$ et $R_5$ ont les significations précédentes ;
p) Les radicaux de formule $-R_6-O-R_7-O-CO-R_8-$ dans laquelle $R_6$, $R_7$, $R_8$, identiques ou différents, représentent des radicaux alkylènes ayant de 2 à 6 atomes de carbone et le radical $R_7$ est substitué par un groupement hydroxyle ;

B) U représente -O- ou $-NR_9-$ où $R_9$ est un radical alkyle, linéaire ou ramifié, ayant de 1 à 4 atomes de carbone;
C) R2 représente un radical méthyle;

- V est choisi parmi: un radical alkyle, linéaire ou ramifié, ayant 5 à 18 atomes de carbone; un radical de formule $-(CH_2)_{10}-COO-R_{12}$ dans laquelle $R_{12}$ représente un radical alkyle, linéaire de 1 à 6 atomes de carbone, un radical de formule $-(CH_2)_3-O-R_{13,}$ $R_{13}$ représente un atome d'hydrogène, un oxyde d'éthylène, un groupement mono ou polyoxyéthyléné, un groupement mono ou polyoxypropyléné, un enchaînement mixte constitué de motifs d'oxyde d'éthylène et d'oxyde de propylène ou un groupement acyle ayant de 2 à 6 atomes de carbone.

- 

**14.** Composition selon la revendication 8, **caractérisée par le fait que** la silicone aminée est un polydiorganosiloxane linéaire de formule (V) présentant les valeurs suivantes pour les symboles r, s et t :

- r est un nombre entier ou fractionnaire allant de 0 à 98 avec la condition selon laquelle si r = 0, au moins un des deux radicaux Y représente le radical Z
- s est un nombre entier ou fractionnaire allant de 0 à 48 ;
- t est un nombre entier ou fractionnaire allant de 0 à 1598 ;
- la somme r + s + t est un nombre entier ou fractionnaire allant de 0 à 1598.

**15.** Composition selon l'une quelconque des revendications 1 à 14, **caractérisée par le fait que** la silicone aminée est un polyorganosiloxane ayant la structure suivante :

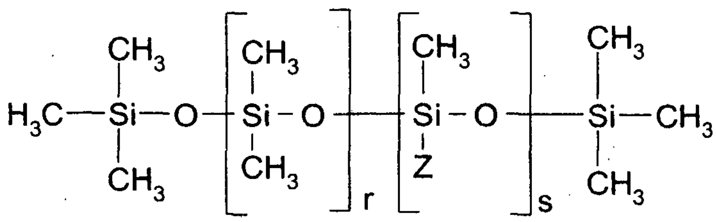

avec Z =

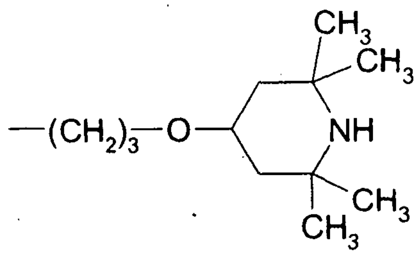

s varie de 1 à 50, de préférence de 2 à 30 ;
r varie de 50 à 2000, de préférence de 500 à 1700.

**16.** Composition selon l'une quelconque des revendications 1 à 15, **caractérisée par le fait que** la silicone aminée est présente dans des concentrations allant de 0,001 % à 10 % en poids, de préférence de 0,01 % à 5 % en poids, et encore plus préférentiellement de 0,1 % à 3 % en poids, du poids total de la composition finale.

**17.** Composition selon l'une quelconque, des revendications 1 à 16, **caractérisée par le fait que** la totalité des tensioactifs est comprise dans des quantités allant de 6 et 35% en poids par rapport au poids total de la composition, de préférence de 8 à 25% en poids.

**18.** Composition selon l'une quelconque des revendications 1 à 17, **caractérisée par le fait que** la composition comprend en outre au moins un polymère cationique.

**19.** Composition selon la revendication 18, **caractérisée par** le fait le polymère cationique est choisi parmi les dérivés d'éther de cellulose quaternaires, les homopolymères de sel de diallyldiméthylammonium et les copolymères de sel de diallyldiméthylammonium et d'acrylamide, les polysaccharides cationiques non cellulosiques, les copolymères quaternaires de vinylpyrrolidone et de sel de vinyl imidazole.

**20.** Composition selon la revendication 18, **caractérisée par** le fait le polymère cationique est choisi parmi les polymères qui sont constitués de motifs récurrents répondant à la formule :

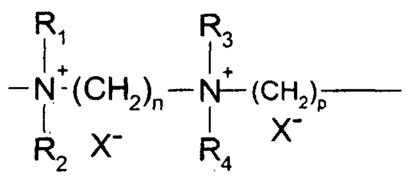
(I)

dans laquelle $R_1$, $R_2$, $R_3$ et $R_4$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.

**21.** Composition selon l'une quelconque des revendications 18 à 20, **caractérisée en ce que** ledit polymère cationique représente de 0,05 % à 10 % en poids, de préférence de 0,1 % à 5 % en poids, et encore plus préférentiellement de 0,25 % à 3 % en poids, du poids total de la composition.

**22.** Composition selon l'une quelconque des revendications 1 à 21, **caractérisée par le fait que** le milieu aqueux cosmétiquement acceptable est constitué uniquement par de l'eau ou par un mélange d'eau et d'un solvant cosmétiquement acceptable.

**23.** Composition selon la revendication 22, **caractérisée par le fait que** les le solvant est choisi les alcools inférieurs en $C_1$-$C_4$, comme l'éthanol, l'isopropanol, le tertiobutanol, le n-butanol ; les alkylèneglycols comme le propylèneglycol ou l'hexylèneglycol, le glycérol.

**24.** Composition selon l'une quelconque des revendications 1 à 23, **caractérisée par le fait qu'**elle contient en plus un ou plusieurs adjuvants choisis par les polymères anioniques ou non ioniques ou amphotères, les protéines, les hydrolysats de protéines, les céramides, les pseudocéramides, les huiles végétales, les acides gras, les hydroxyacides, les vitamines, les provitamines telle que le panthénol, les silicones volatiles ou non volatiles, solubles ou insolubles dans le milieu différentes des silicones aminées stériquement encombrée, les filtres UV, les agents hydratants, les agents antipelliculaires ou antiséborrhéiques, les agents antichute des cheveux, les agents antiradicaux libres, les agents opacifiants et leurs mélanges.

**25.** Utilisation en cosmétique d'une composition telle définie à l'une quelconque des revendications précédentes pour le nettoyage et/ou le soin et/ou le conditionnement et/ou le coiffage des cheveux.

**26.** Utilisation d'une composition telle définie dans l'une quelconque des revendications 1 à 24 pour le nettoyage et/ou le démaquillage des matières kératiniques.

**27.** Procédé de lavage et de conditionnement des matières kératiniques telles que les cheveux consistant à appliquer sur lesdites matières mouillées une quantité efficace d'une composition telle que définie dans l'une quelconque des revendications 1 à 24 puis à effectuer un rinçage à l'eau après un éventuel temps de pause.

## Claims

**1.** Conditioning and detergent cosmetic composition, **characterized in that** it comprises, in an aqueous medium:

(A) at least one anionic surfactant of sulfate and/or sulfonate type,
(B) at least one anionic surfactant of carboxylic type other than the surfactant (A),
(C) at least one amphoteric surfactant,

(D) at least one amino silicone comprising an amine function borne by a sterically hindered group on at least one of its ends and/or on side chains, this group being integrated in a 5- to 8-membered heterocycle, the carbons $\alpha$ and $\alpha'$ to the nitrogen atom being totally substituted with atoms or groups other than hydrogen, the amino silicone being chosen from polyorganosiloxanes containing, per mole, at least one unit of general formula (I):

$$(I) \qquad (R)_a(X)_bZSi(O)_{\frac{3-(a+b)}{2}}$$

in which:

• the symbols R are identical or different and represent a monovalent hydrocarbon-based radical chosen from linear or branched alkyl radicals containing from 1 to 4 carbon atoms, phenyl, benzyl and 3,3,3-trifluoropropyl;
• the symbols X are identical or different and represent a monovalent radical chosen from a hydroxyl group and a linear or branched alkoxy radical containing from 1 to 3 carbon atoms;
• Z represents a monovalent radical comprising an amine function borne by a sterically hindered group, namely one or more sterically hindered piperidyl group(s) chosen from:

3) the radicals of formula (II):

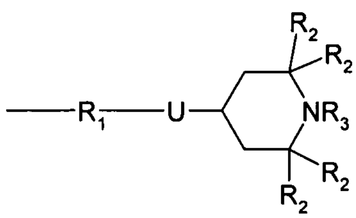

(II)

in which:

A) $R_1$ is a divalent hydrocarbon-based radical optionally comprising one or more heteroatoms such as O and N, chosen from:

h) linear or branched alkylene radicals containing from 2 to 18 carbon atoms;

i) alkylene-carbonyl radicals in which the linear or branched alkylene portion contains 2 to 20 carbon atoms;
j) alkylene-cyclohexylene radicals in which the linear or branched alkylene portion contains 1 to 12 carbon atoms and the cyclohexylene portion comprises an OH group and optionally one or two alkyl radicals containing from 1 to 4 carbon atoms;
k) the radicals of formula $-R_4-O-R_5-$ in which the radicals $R_4$ and $R_5$, which may be identical or different, represent alkylene radicals containing 1 to 12 carbon atoms;
l) the radicals of formula $-R_4-O-R_5-$ in which the radicals $R_4$ and $R_5$ have the same meanings as above and one or both of them is (are) substituted with one or two OH group(s);
m) the radicals of formulae $-R_4-COO-R_5-$ and $-R_4-OCO-R_5-$ in which $R_4$ and $R_5$ have the above meanings;
n) the radicals of formula $-R_6-O-R_7-O-CO-R_8-$ in which $R_6$, $R_7$ and $R_8$, which may be identical or different, represent alkylene radicals containing from 2 to 12 carbon atoms and the radical $R_7$ is optionally substituted with a hydroxyl group;

B) U represents -O- or $-NR_9-$, $R_9$ being a monovalent radical chosen from a hydrogen atom, a linear or branched alkyl radical containing from 1 to 6 carbon atoms; a divalent radical $-R_1-$ having the meaning given above, one of the valency bonds being linked to the nitrogen atom of $-NR_9-$, the other being linked to a silicon atom;
C) $R_2$ are identical or different radicals chosen from linear or branched alkyl radicals containing from 1 to 3 carbon atoms and phenyl;

D) $R_3$ represents a hydrogen atom or a radical $R_2$;

- a is a number chosen from 0, 1 and 2,
- b is a number chosen from 0, 1 and 2;
- the sum a + b is not more than 2,

the sulfate or sulfonate anionic surfactant/carboxylic anionic surfactant weight ratio ranging from 2 to 25.

**2.** Composition according to Claim 1, **characterized in that** the sulfate and/or sulfonate anionic surfactants are salts of alkyl sulfates, alkylamido sulfates, alkyl ether sulfates, alkylamido ether sulfates, alkylaryl ether sulfates, alkyl ether sulfosuccinates, acyl isethionates or methyl acyl taurates; the alkyl or acyl radical of all these various compounds preferably containing from 8 to 24 carbon atoms, and the aryl radical preferably denoting a phenyl or benzyl group.

**3.** Composition according to either of Claims 1 and 2, **characterized in that** the anionic surfactants of carboxylic type other than the surfactants (A) are chosen from alkyl D-galactoside uronic acids and salts thereof, polyoxyalkylenated ($C_6$-$C_{24}$)alkyl ether carboxylic acids and salts thereof, polyoxyalkylenated ($C_6$-$C_{24}$)alkylaryl ether carboxylic acids and salts thereof, polyoxyalkylenated ($C_6$-$C_{24}$)alkylamido ether carboxylic acids and salts thereof, ($C_6$-$C_{24}$)acyl sarcosinates and salts thereof, ($C_6$-$C_{24}$)acyl lactylates and salts thereof and ($C_6$-$C_{24}$)acyl glutamates and salts thereof, ($C_6$-$C_{24}$)alkylpolyglycoside carboxylic acid esters and salts thereof.

**4.** Composition according to any one of Claims 1 to 3, **characterized in that** the amphoteric and/or zwitterionic surfactants are aliphatic secondary or tertiary amine derivatives, in which the aliphatic radical is a linear or branched chain containing 8 to 22 carbon atoms and containing at least one water-solubilizing anionic group or ($C_8$-$C_{20}$)alkyl betaines, sulfobetaines, ($C_8$-$C_{20}$)alkylamido($C_1$-$C_6$)alkyl betaines or ($C_8$-$C_{20}$)alkylamido($C_1$-$C_6$)alkyl sulfobetaines.

**5.** Composition according to any one of Claims 1 to 4, **characterized in that** the sulfate and/or sulfonate anionic surfactant(s) is (are) present in concentrations ranging from 2.5% to 25% by weight relative to the total weight of the composition.

**6.** Composition according to any one of Claims 1 to 5, **characterized in that** the said carboxylic anionic surfactant(s) is (are) present in concentrations ranging from 0.1% to 15% by weight and preferably from 0.5% to 10% by weight relative to the total weight of the composition.

**7.** Composition according to any one of Claims 1 to 6, **characterized in that** the amphoteric surfactant(s) is (are) present in concentrations ranging from 0.1% to 20% by weight and preferably from 1% to 15% by weight relative to the total weight of the composition.

**8.** Composition according to any one of Claims 1 to 7, **characterized in that** the sulfate or sulfonate anionic surfactant/amphoteric surfactant weight ratio ranges from 0.5 to 12, more particularly from 1 to 10 and even more preferentially from 1.5 to 5.

**9.** Composition according to any one of Claims 1 to 8, **characterized in that** the carboxylic anionic surfactant/amphoteric surfactant weight ratio ranges from 0.3 to 20 and preferably from 0.5 to 15.

**10.** Composition according to any one of Claims 1 to 9, **characterized in that** the amino silicone also comprises at least one other siloxyl unit of formula (III):

$$\text{(III)} \qquad (R)_c(X)_dVSi(O)_{\frac{3-(c+d)}{2}}$$

in which:

- the symbols R and X have the same meanings as those given above with respect to formula (I);
- the symbol V represents: a linear or branched alkyl radical containing from 5 to 20 carbon atoms; a radical of formula $-(CH_2)_p-COOR_{12}$ in which p represents a number from 5 to 20 and $R_{12}$ represents a linear or branched alkyl radical of 1 to 12 carbon atoms; a radical of formula $-(CH_2)_q-O-R_{13}$ in which q represents a number from

3 to 10 and $R_{13}$ represents a hydrogen atom, an ethylene oxide, a mono- or polyoxyethylene group, a mono- or polyoxypropylene group, a mixed chain consisting of ethylene oxide and propylene oxide units or an acyl radical containing from 2 to 12 carbon atoms;

- c is a number chosen from 0, 1 and 2;
- d is a number chosen from 0, 1 and 2;
- the sum c + d is not more than 2.

**11.** Composition according to any one of Claims 1 to 10, **characterized in that** the amino silicone also comprises (an) other siloxyl unit(s) of formula (IV):

$$\text{(IV)} \qquad (R)_e(X)_f Si(O)_{\frac{4-(e+f)}{2}}$$

in which

- R and X have the same meanings as those given with respect to formula (I);
- e is a number chosen from 0, 1, 2 and 3;
- f is a number chosen from 0, 1, 2 and 3;
- the sum e + f is not more than 3.

**12.** Composition according to any one of Claims 1 to 11, **characterized in that** the amino silicone is a linear polyorganosiloxane of mean formula (V):

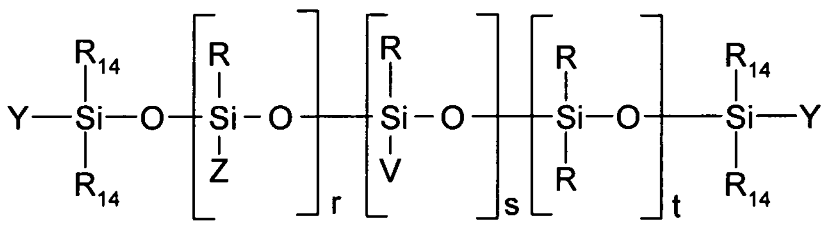

(V)

in which:

- the symbols R, Z and V have the meanings given above with respect to formulae (I) and (III);
- the symbol Y represents a monovalent radical chosen from the radicals R, Z, V and X;
- the symbols $R_{14}$ are identical or different and represent a monovalent radical chosen from a radical R and a radical X as defined above with respect to formula (I);
- r, s and t are equal to zero or represent integers or fractional numbers greater than zero, with the additional condition that if r = 0, at least one of the two radicals Y represents the radical Z.

**13.** Composition according to any one of Claims 1 to 12, **characterized in that** the amino silicone is a polyorganosiloxane containing units of formulae (I) + optionally (III) + optionally (IV) or a polyorganosiloxane of formula (V):

these polyorganosiloxanes containing on average, per mole: from 2 to 1600 silicon atoms, from 1 to 100 radicals Z such as those defined below and from 0 to 50 radicals V such as those defined below;

- and in the structure of which:

  - R is chosen from methyl, ethyl, n-propyl and isopropyl radicals, preferably methyl;
  - X is chosen from hydroxyl, methoxy and ethoxy radicals;
  - Z is chosen from radicals containing piperidyl group(s):

2) of formula (II) in which:

A) $R_1$ is a divalent hydrocarbon-based radical optionally comprising one or more heteroatoms such as O and N, chosen from:

i) linear or branched alkylene radicals containing from 2 to 18 carbon atoms;
j) linear or branched alkylene radicals containing from 3 to 12 carbon atoms;
k) the radical-$(CH_2)_{10}$-CO-;
l) alkylene-cyclohexylene radicals in which the linear or branched alkylene portion contains from 2 to 6 carbon atoms and the cyclohexylene portion contains an -OH group and optionally one or two alkyl substituents containing 1 to 4 carbon atoms;
m) the radicals of formula -$R_4$-O-$R_5$- in which the radicals $R_4$ and $R_5$, which may be identical or different, represent alkylene radicals containing from 2 to 6 carbon atoms;
n) the radicals of formula -$R_4$-O-$R_5$- in which the radicals $R_4$ and $R_5$ have the meanings given above and $R_5$ is substituted with an OH group;
o) the radicals of formulae -$R_4$-COO-$R_5$- and -$R_4$-OCO-$R_5$- in which $R_4$ and $R_5$ have the above meanings;
p) the radicals of formula -$R_6$-O-$R_7$-O-CO-$R_8$- in which $R_6$, $R_7$ and $R_8$, which may be identical or different, represent alkylene radicals containing from 2 to 6 carbon atoms and the radical $R_7$ is substituted with a hydroxyl group;

B) U represents -O- or -$NR_9$-, in which $R_9$ is a linear or branched alkyl radical containing from 1 to 4 carbon atoms;
C) $R_2$ represents a methyl radical;

• V is chosen from: a linear or branched alkyl radical containing from 5 to 18 carbon atoms; a radical of formula -$(CH_2)_{10}$-COO-$R_{12}$ in which $R_{12}$ represents a linear alkyl radical of 1 to 6 carbon atoms, a radical of formula -$(CH_2)_3$-O-$R_{13}$, in which $R_{13}$ represents a hydrogen atom, an ethylene oxide, a mono- or polyoxyethylene group, a mono- or polyoxypropylene group, a mixed chain consisting of ethylene oxide and propylene oxide units or an acyl group containing from 2 to 6 carbon atoms.

**14.** Composition according to Claim 8, **characterized in that** the amino silicone is a linear polydiorganosiloxane of formula (V) having the following values for the symbols r, s and t:

• r is an integer or fractional number ranging from 0 to 98, with the condition that if r = 0, at least one of the two radicals Y represents the radical Z;
• s is an integer or fractional number ranging from 0 to 48;
• t is an integer or fractional number ranging from 0 to 1598;
• the sum r + s + t is an integer or fractional number ranging from 0 to 1598.

**15.** Composition according to any one of Claims 1 to 14, **characterized in that** the amino silicone is a polyorganosiloxane having the following structure:

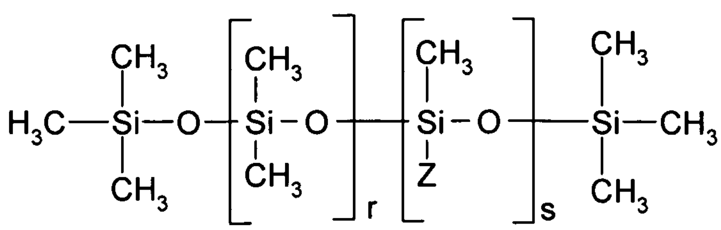

with Z =

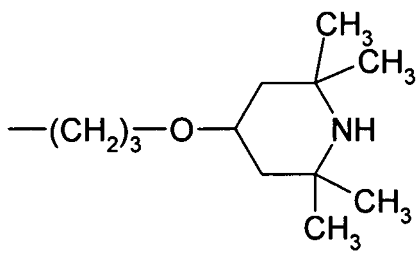

s ranges from 1 to 50 and preferably from 2 to 30,
r ranges from 50 to 2000 and preferably from 500 to 1700.

**16.** Composition according to any one of Claims 1 to 15, **characterized in that** the amino silicone is present in concentrations ranging from 0.001% to 10% by weight, preferably from 0.01% to 5% by weight and even more preferentially from 0.1% to 3% by weight relative to the total weight of the final composition.

**17.** Composition according to any one of Claims 1 to 16, **characterized in that** the total amount of surfactants is in amounts ranging from 6% to 35% by weight and preferably from 8% to 25% by weight relative to the total weight of the composition.

**18.** Composition according to any one of Claims 1 to 17, **characterized in that** the composition also comprises at least one cationic polymer.

**19.** Composition according to Claim 18, **characterized in that** the cationic polymer is chosen from quaternary cellulose ether derivatives, diallyldimethylammonium salt homopolymers, and copolymers of a diallyldimethylammonium salt and of acrylamide, non-cellulose-based cationic polysaccharides, and quaternary copolymers of vinylpyrrolidone and of a vinylimidazole salt.

**20.** Composition according to Claim 18, **characterized in that** the cationic polymer is chosen from polymers consisting of repeating units corresponding to the formula:

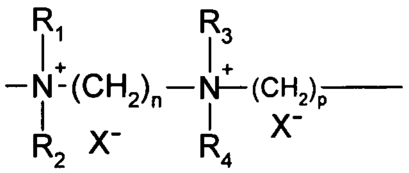

(I)

in which $R_1$, $R_2$, $R_3$ and $R_4$, which may be identical or different, denote an alkyl or hydroxyalkyl radical containing from 1 to 4 carbon atoms approximately, n and p are integers ranging from 2 to 20 approximately, and $X^-$ is an anion derived from a mineral or organic acid.

**21.** Composition according to any one of Claims 18 to 20, **characterized in that** the said cationic polymer represents from 0.05% to 10% by weight, preferably from 0.1% to 5% by weight and even more preferentially from 0.25% to 3% by weight relative to the total weight of the composition.

**22.** Composition according to any one of Claims 1 to 21, **characterized in that** the cosmetically acceptable aqueous medium consists solely of water or of a mixture of water and of a cosmetically acceptable solvent.

**23.** Composition according to Claim 22, **characterized in that** the solvent is chosen from $C_1$-$C_4$ lower alcohols, for instance ethanol, isopropanol, tert-butanol or n-butanol; alkylene glycols, for instance propylene glycol or hexylene glycol, and glycerol.

**24.** Composition according to any one of Claims 1 to 23, **characterized in that** it also contains one or more adjuvants chosen from anionic, nonionic or amphoteric polymers, proteins, protein hydrolysates, ceramides, pseudoceramides, plant oils, fatty acids, hydroxy acids, vitamins, provitamins such as panthenol, volatile and non-volatile silicones

which are soluble or insoluble in the medium and are different from the sterically hindered aminosilicones, UV-screening agents, moisturizers, antidandruff or anti-seborrhoeic agents, hair-loss counteractants, free-radical scavengers and opacifiers, and mixtures thereof.

**25.** Cosmetic use of a composition as defined in any one of the preceding claims, for cleansing and/or caring for and/or conditioning and/or styling the hair.

**26.** Use of a composition as defined in any one of Claims 1 to 24, for cleansing and/or removing makeup from keratin materials.

**27.** Process for washing and conditioning keratin materials such as the hair, which consists in applying to the said moistened materials an effective amount of a composition as defined in any one of Claims 1 to 24, and then in rinsing it out with water, after an optional leave-in time.

**Patentansprüche**

**1.** Konditionierende und reinigende, kosmetische Zusammensetzung, **dadurch gekennzeichnet, dass** sie in einem wässrigen Medium enthält:

(A) mindestens einen anionischen grenzflächenaktiven Stoff vom Typ Sulfat und/oder Sulfonat,
(B) mindestens einen anionischen grenzflächenaktiven Stoff vom Carboxytyp, der von dem grenzflächenaktiven Stoff (A) verschieden ist,
(C) mindestens einen amphoteren grenzflächenaktiven Stoff, und
(D) mindestens ein aminiertes Silicon, das an mindestens einem seiner Enden und/oder an Seitenketten eine von einer sterisch gehinderten Gruppe getragene Aminofunktion aufweist, wobei diese Gruppe in einen 5- bis 8-gliedrigen Heterocyclus integriert ist und die Kohlenstoffatome in α- und α'-Stellung des Stickstoffatoms vollständig mit Atomen oder Gruppen substituiert sind, die von Wasserstoff verschieden sind; wobei das aminierte Silicon unter den Polyorganosiloxanen ausgewählt ist, die pro Mol mindestens eine Einheit der folgenden allgemeinen Formel (I) aufweisen:

$$\text{(I)} \qquad (R)_a(X)_bZSi(O)_{\frac{3-(a+b)}{2}}$$

in der Formel:

■ die Symbole R sind gleich oder verschieden und bedeuten eine einwertige Kohlenwasserstoffgruppe, die unter den linearen oder verzweigten Alkylgruppen mit 1 bis 4 Kohlenstoffatomen, Phenyl, Benzyl und 3,3,3-Trifluorpropyl ausgewählt ist;
■ die Symbole X sind gleich oder verschieden und bedeuten eine einwertige Gruppe, die unter einer Hydroxygruppe und einer linearen oder verzweigten Alkoxygruppe mit 1 bis 3 Kohlenstoffatomen ausgewählt ist;
■ Z ist eine einwertige Gruppe, die eine Aminofunktion aufweist, die von einer sterisch gehinderten Gruppe getragen wird, d.h. eine oder mehrere sterisch gehinderte Piperidinylgruppe(n), die ausgewählt sind unter:

den Gruppen der folgenden Formel (II):

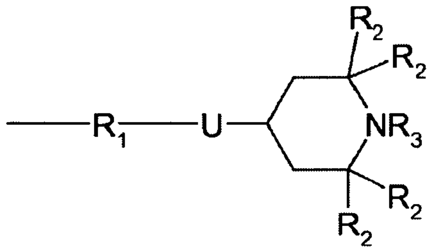

(II)

in der Formel:

A) $R_1$ ist eine zweiwertige Kohlenwasserstoffgruppe, die gegebenenfalls ein oder mehrere Heteroatome wie O und N enthält und die ausgewählt ist unter:

a) linearen oder verzweigten Alkylengruppen mit 2 bis 18 Kohlenstoffatomen;
b) Alkylen-carbonylgruppen, deren linearer oder verzweigter Alkylenteil 2 bis 20 Kohlenstoffatome aufweist;
c) Alkylen-cyclohexylengruppen, deren linearer oder verzweigter Alkylenteil 1 bis 12 Kohlenstoffatome aufweist und deren Cyclohexylenteil eine OH-Gruppe und gegebenenfalls 1 oder 2 Alkylgruppen mit 1 bis 4 Kohlenstoffatomen umfasst;
d) den Gruppen der Formel $-R_4-O-R_5-$, worin die Gruppen $R_4$ und $R_5$, die gleich oder verschieden sind, Alkylengruppen mit 1 bis 12 Kohlenstoffatomen bedeuten;
e) den Gruppen der Formel $-R_4-O-R_5-$, worin die Gruppen $R_4$ und $R_5$ die oben angegebenen Bedeutungen aufweisen, wobei eine Gruppe oder beide Gruppen mit einer oder mit zwei Gruppe(n) OH substituiert sind;
f) den Gruppen der Formel $-R_4-COO-R_5-$ und $-R_4-OCO-R_5$, worin die Gruppen $R_4$ und $R_5$ die oben angegebenen Bedeutungen aufweisen;
g) den Gruppen der Formel $-R_6-O-R_7-O-CO-R_8-$, worin die Gruppen $R_6$, $R_7$ und $R_8$, die gleich oder verschieden sind, Alkylengruppen mit 2 bis 12 Kohlenstoffatomen bedeuten, wobei die Gruppe $R_7$ gegebenenfalls mit einer Hydroxygruppe substituiert ist;

B) U bedeutet -O- oder $-NR_9-$, wobei $R_9$ eine einwertige Gruppe ist, die ausgewählt ist unter einem Wasserstoffatom, einer geradkettigen oder verzweigten Alkylgruppe mit 1 bis 6 Kohlenstoffatomen; einer zweiwertigen Gruppe $-R_1-$ mit den oben angegebenen Bedeutungen, wobei eine Bindung zu dem Stickstoffatom von $-NR_9-$ geht und die andere an ein Siliciumatom gebunden ist;
C) $R_2$ sind Gruppen, die gleich oder verschieden sind und die unter den geradkettigen oder verzweigten Alkylgruppen mit 1 bis 3 Kohlenstoffatomen und Phenyl ausgewählt sind;
D) $R_3$ bedeutet ein Wasserstoffatom oder die Gruppe $R_2$;

■ a ist eine Zahl, die unter 0, 1 und 2 ausgewählt ist;
■ b ist eine Zahl, die unter 0, 1 und 2 ausgewählt ist;
■ die Summe a + b ist höchstens 2.

wobei das Gewichtsverhältnis des anionischen
grenzflächenaktiven Sulfats oder Sulfonats und des anionischen grenzflächenaktiven Stoffes vom Carboxytyp im Bereich von 2 bis 25 liegt.

**2.** Zusammensetzung nach Anspruch 1, **dadurch gekennzeichnet, dass** es sich bei den anionischen grenzflächenaktiven Sulfaten und/oder Sulfonaten um die Salze der folgenden Verbindungen handelt: Alkylsulfate, Alkylamidosulfate, Alkylethersulfate, Alkylamidoethersulfate, Alkylarylethersulfate, Alkylethersulfosuccinate, Acylisethionate und Methylacyltaurate, wobei die Alkyl- oder Acylgruppen der verschiedenen genannten Verbindungen vorzugsweise 8 bis 24 Kohlenstoffatome aufweisen und die Arylgruppe vorzugsweise Phenyl oder Benzyl bedeutet.

**3.** Zusammensetzung nach einem der Ansprüche 1 oder 2, **dadurch gekennzeichnet, dass** die anionischen grenzflächenaktiven Stoffe vom Carboxytyp, die von den grenzflächenaktiven Stoffen (A) verschieden sind, unter den Alkyl-D-galactosiduronsäuren und ihren Salzen, polyalkoxylierten Alkyl($C_{6-24}$)ethercarbonsäuren und ihren Salzen,

polyalkoxylierten Alkyl($C_{6-24}$)arylethercarbonsäuren und ihren Salzen, polyalkoxylierten Alkyl($C_{6-24}$)amidoethercarbonsäuren und ihren Salzen, Acyl($C_{6-24}$)sarcosinaten und ihren Salzen, Acyl($C_{6-24}$)lactylaten und ihren Salzen und Acyl($C_{6-24}$)glutamaten und ihren Salzen ausgewählt sind.

**4.** Zusammensetzung nach einem der Ansprüche 1 bis 3, **dadurch gekennzeichnet, dass** die amphoteren und/oder zwitterionischen grenzflächenaktiven Stoffe aliphatische, sekundäre oder tertiäre Aminderivate, bei denen die aliphatische Gruppe eine geradkettige oder verzweigte Kette mit 8 bis 22 Kohlenstoffatomen ist, die mindestens eine Wasserlöslichkeit vermittelnde anionische Gruppe trägt, oder Alkyl($C_{8-20}$)betaine, Sulfobetaine, Alkyl($C_{8-20}$)amidoalkyl($C_{1-6}$)betaine oder Alkyl($C_{8-20}$)amidoalkyl($C_{1-6}$)sulfobetaine sind.

**5.** Zusammensetzung nach einem der Ansprüche 1 bis 4, **dadurch gekennzeichnet, dass** das oder die anionische (n) grenzflächenaktive(n) Sulfat(e) und/oder Sulfonat(e) in Konzentrationen von 2,5 bis 25 Gew.-%, bezogen auf das Gesamtgewicht der Zusammensetzung, enthalten ist (sind).

**6.** Zusammensetzung nach einem der Ansprüche 1 bis 5, **dadurch gekennzeichnet, dass** die anionische(n) grenzflächenaktive(n) Carboxyverbindung(en) in Konzentrationen von 0,1 bis 15 Gew.-% und vorzugsweise 0,5 bis 10 Gew.-%, bezogen auf das Gesamtgewicht der Zusammensetzung, enthalten ist (sind).

**7.** Zusammensetzung nach einem der Ansprüche 1 bis 6, **dadurch gekennzeichnet, dass** der oder die amphotere (n) grenzflächenaktive(n) Stoff(e) in Konzentrationen von 0,1 bis 20 Gew.-% und vorzugsweise 1 bis 15 Gew.-%, bezogen auf das Gesamtgewicht der Zusammensetzung, enthalten ist (sind).

**8.** Zusammensetzung nach einem der Ansprüche 1 bis 7, **dadurch gekennzeichnet, dass** das Gewichtsverhältnis des anionischen grenzflächenaktiven Sulfats oder Sulfonats und des amphoteren grenzflächenaktiven Stoffes im Bereich von 0,5 bis 12, insbesondere im Bereich von 1 bis 10 und noch bevorzugter im Bereich von 1,5 bis 5 liegt.

**9.** Zusammensetzung nach einem der Ansprüche 1 bis 8, **dadurch gekennzeichnet, dass** das auf das Gewicht bezogene Verhältnis anionische grenzflächenaktive Carboxyverbindung/amphoterer grenzflächenaktiver Stoff im Bereich von 0,3 bis 20 und vorzugsweise im Bereich von 0,5 bis 15 liegt.

**10.** Zusammensetzung nach einem der Ansprüche 1 bis 9, **dadurch gekennzeichnet, dass** das aminierte Silicon ferner mindestens eine weitere Siloxyeinheit der Formel (III) enthält:

$$\text{(III)} \qquad (R)_c(X)_dVSi(O)_{\frac{3-(c+d)}{2}}$$

in der Formel:

- die Symbole R und X weisen die in Bezug auf die Formel (I) angegebenen Bedeutungen auf:
  - das Symbol V bedeutet: eine geradkettige oder verzweigte Alkylgruppe mit 5 bis 20 Kohlenstoffatomen; eine Gruppe der Formel $-(CH_2)_p-COOR_{12}$, worin p eine Zahl von 5 bis 20 bedeutet und $R_{12}$ eine geradkettige oder verzweigte Alkylgruppe mit 1 bis 12 Kohlenstoffatomen ist; eine Gruppe der Formel $-(CH_2)_q-O-R_{13}$, worin q eine Zahl von 3 bis 10 bedeutet und $R_{13}$ ein Wasserstoffatom, Ethylenoxid, eine mono- oder polyethoxylierte Gruppe, eine mono- oder polypropoxylierte Gruppe, eine gemischte Gruppierung, die aus Ethylenoxideinheiten und Propylenoxideinheiten gebildet ist, oder eine Acylgruppe mit 2 bis 12 Kohlenstoffatomen;
  - c ist eine Zahl, die unter 0, 1 und 2 ausgewählt ist;
  - d ist eine Zahl, die unter 0, 1 und 2 ausgewählt ist;
  - die Summe c + d ist höchstens 2.

**11.** Zusammensetzung nach einem der Ansprüche 1 bis 10, **dadurch gekennzeichnet, dass** das aminierte Silicon ferner (eine) weitere Siloxyeinheit(en) der Formel (IV) enthält:

$$\text{(IV)} \qquad (R)_e(X)_f Si(O)_{\frac{4-(e+f)}{2}}$$

in der Formel:

■ die Symbole R und X weisen die in Bezug auf die Formel (I) angegebenen Bedeutungen auf:

■ e ist eine Zahl, die unter 0, 1, 2 und 3 ausgewählt ist;
■ f ist eine Zahl, die unter 0, 1, 2 und 3 ausgewählt ist;
■ die Summe e + f ist höchstens 3.

**12.** Zusammensetzung nach einem der Ansprüche 1 bis 11, **dadurch gekennzeichnet, dass** das aminierte Silicon ein lineares Polyorganosiloxan der mittleren Formel (V) ist:

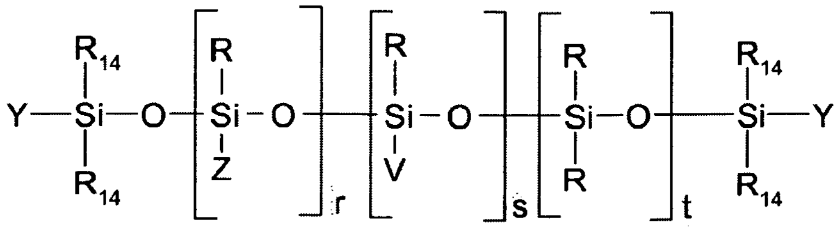

(V)

in der Formel:

■ die Symbole R, Z und V weisen die in Bezug auf die Formeln (I) und (III) angegebenen Bedeutungen auf;
■ das Symbol Y bedeutet eine einwertige Gruppe, die unter den Gruppen R, Z, V und X ausgewählt ist;
■ die Symbole $R_{14}$ sind gleich oder verschieden und bedeuten eine einwertige Gruppe, die unter einer Gruppe R und einer Gruppe X ausgewählt sind, wie sie oben in Bezug auf die Formel (I) definiert sind;
■ r, s und t sind Null oder sie bedeuten ganze oder rationale Zahlen über Null, mit der weiteren Maßgabe, dass mindestens eine der beiden Gruppen Y die Gruppe Z bedeutet, wenn r = 0.

**13.** Zusammensetzung nach einem der Ansprüche 1 bis 12, **dadurch gekennzeichnet, dass** das aminierte Silicon ein Polyorganosiloxan mit Einheit(en) der Formeln (I) + gegebenenfalls (III) + gegebenenfalls (IV) oder ein Polyorganosiloxan der Formel (V) bedeutet:
wobei diese Polyorganosiloxane im Mittel pro Mol aufweisen: 2 bis 1600 Siliciumatome, 1 bis 100 wie oben definierte Gruppen Z und 0 bis 50 wie oben definierte Gruppen V;
und in ihrer Struktur:

• R ist unter den Gruppen Methyl, Ethyl, n-Propyl und Isopropyl und vorzugsweise Methyl ausgewählt;
• X ist unter den Gruppen Hydroxy, Methoxy und Ethoxy ausgewählt;
• Z ist unter den Gruppen mit Piperidinylgruppe(n) der Formel (II) ausgewählt, in der:

A) $R_1$ ist eine zweiwertige Kohlenwasserstoffgruppe, die gegebenenfalls ein oder mehrere Heteroatome wie O und N enthält und die ausgewählt ist unter:

a) linearen oder verzweigten Alkylengruppen mit 2 bis 18 Kohlenstoffatomen;
b) linearen oder verzweigten Alkylengruppen mit 3 bis 12 Kohlenstoffatomen;
c) der Gruppe $(CH_2)_{10}$-CO;
d) Alkylen-cyclohexylengruppen, deren linearer oder verzweigter Alkylenteil 2 bis 6 Kohlenstoffatome aufweist und deren Cyclohexylenteil eine OH-Gruppe und gegebenenfalls 1 oder 2 Alkylsubstituenten mit 1 bis 4 Kohlenstoffatomen aufweist;

e) den Gruppen der Formel $-R_4-O-R_5-$, worin die Gruppen $R_4$ und $R_5$, die gleich oder verschieden sind, Alkylengruppen mit 2 bis 6 Kohlenstoffatomen bedeuten;
f) den Gruppen der Formel $-R_4-O-R_5-$, worin die Gruppen $R_4$ und $R_5$ die oben angegebenen Bedeutungen aufweisen, wobei $R_5$ mit einer OH-Gruppe substituiert
g) den Gruppen der Formel $-R_4-COO-R_5-$ und $-R_4-OCO-R_5$, worin die Gruppen $R_4$ und $R_5$ die oben angegebenen Bedeutungen aufweisen;
h) den Gruppen der Formel $-R_6-O-R_7-O-CO-R_8-$, worin die Gruppen $R_6$, $R_7$ und $R_8$, die gleich oder verschieden sind, Alkylengruppen mit 2 bis 6 Kohlenstoffatomen bedeuten und die Gruppe $R_7$ mit einer Hydroxygruppe substituiert ist;

B) U bedeutet -O- oder $-NR_9-$, wobei $R_9$ eine geradkettige oder verzweigte Alkylgruppe mit 1 bis 4 Kohlenstoffatomen ist; C) $R_2$ bedeutet Methyl;

■ V ist ausgewählt unter: einer geradkettigen oder verzweigten Alkylgruppe mit 5 bis 18 Kohlenstoffatomen; einer Gruppe der Formel $-(CH_2)_{10}-COO-R_{12}$, worin $R_{12}$ eine geradkettige Alkylgruppe mit 1 bis 6 Kohlenstoffatomen ist; einer Gruppe der Formel $-(CH_2)_3-O-R_{13}$, worin $R_{13}$ ein Wasserstoffatom, eine Ethylenoxidgruppe, eine mono- oder polyethoxylierte Gruppe, eine mono- oder polypropoxylierte Gruppe, eine gemischte Gruppierung, die aus Ethylenoxideinheiten und Propylenoxideinheiten gebildet ist, oder eine Acylgruppe mit 2 bis 6 Kohlenstoffatomen ist.

**14.** Zusammensetzung nach Anspruch 8, **dadurch gekennzeichnet, dass** das aminierte Silicon ein lineares Polyorganosiloxan der Formel (V) ist, das für die Symbole r, s und t die folgenden Werte aufweist:

■ r ist eine ganze oder rationale Zahl im Bereich von 0 bis 98, mit der Maßgabe, dass mindestens eine der beiden Gruppen Y die Gruppe Z bedeutet, wenn r = 0;
■ s ist eine ganze oder rationale Zahl im Bereich von 0 bis 48;
■ t ist eine ganze oder rationale Zahl im Bereich von 0 bis 1598;
■ die Summe r + s + t ist eine ganze oder rationale Zahl im Bereich von 0 bis 1598.

**15.** Zusammensetzung nach einem der Ansprüche 1 bis 14, **dadurch gekennzeichnet, dass** das aminierte Silicon ein Polyorganosiloxan mit der folgenden Struktur ist:

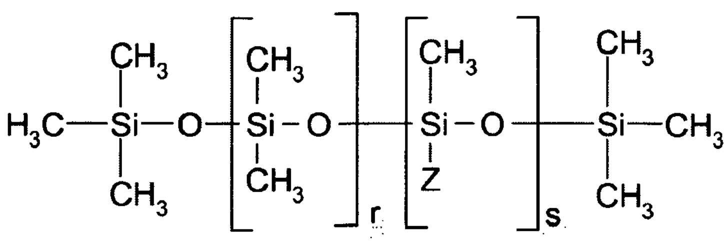

mit Z =

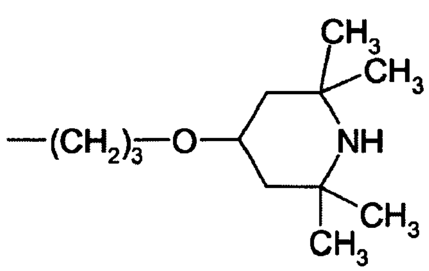

s liegt im Bereich von 1 bis 50 und vorzugsweise 2 bis 30;
r liegt im Bereich von 5 bis 2000 und vorzugsweise 500 bis 1700.

**16.** Zusammensetzung nach einem der Ansprüche 1 bis 15, **dadurch gekennzeichnet, dass** das aminierte Silicon in Konzentrationen von 0,001 bis 10 Gew.-%, vorzugsweise 0,01 bis 5 Gew.-% und noch bevorzugter 0, 1 bis 3 Gew.-%, bezogen auf das Gesamtgewicht der fertigen Zusammensetzung, enthalten ist.

**17.** Zusammensetzung nach einem der Ansprüche 1 bis 16, **dadurch gekennzeichnet, dass** die Gesamtmenge der grenzflächenaktiven Stoffe 6 bis 35 Gew.-%, bezogen auf das Gesamtgewicht der fertigen Zusammensetzung, und vorzugsweise 8 bis 25 Gew.-% ausmacht.

**18.** Zusammensetzung nach einem der Ansprüche 1 bis 17, **dadurch gekennzeichnet, dass** die Zusammensetzung ferner mindestens ein kationisches Polymer enthält.

**19.** Zusammensetzung nach Anspruch 18, **dadurch gekennzeichnet, dass** das kationische Polymer unter den quartären Celluloseetherderivaten, Homopolymeren von Diallyldimethylammoniumsalzen, Copolymeren von Diallyldimethylammoniumsalzen und Acrylamid, kationischen Polysacchariden, die nicht auf Cellulose basieren, quartären Copolymeren von Vinylpyrrolidon und einem Vinylimidazolsalz ausgewählt ist.

**20.** Zusammensetzung nach Anspruch 18, **dadurch gekennzeichnet, dass** das kationische Polymer unter den Polymeren ausgewählt ist, die Wiederholungseinheiten der folgenden Formel enthalten:

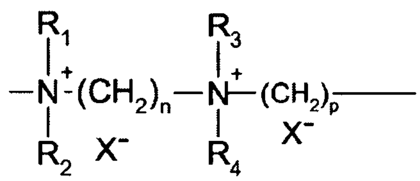 (I)

worin die Gruppen $R_1$, $R_2$, $R_3$ und $R_4$, die gleich oder verschieden sind, eine Alkyl- oder Hydroxyalkylgruppe mit etwa 1 bis 4 Kohlenstoffatomen bedeuten, n und p ganze Zahlen im Bereich von etwa 2 bis 20 sind und X- ein von einer anorganischen oder organischen Säure abgeleitetes Anion ist.

**21.** Zusammensetzung nach einem der Ansprüche 18 bis 20, **dadurch gekennzeichnet, dass** das kationische Polymer 0,05 bis 10 Gew.-%, vorzugsweise 0, 1 bis 5 Gew.-% und noch bevorzugter 0,25 bis 3 Gew.-% des Gesamtgewichts der Zusammensetzung ausmacht.

**22.** Zusammensetzung nach einem der Ansprüche 1 bis 21, **dadurch gekennzeichnet, dass** das kosmetisch akzeptable wässrige Medium nur aus Wasser oder aus einem Gemisch von Wasser und einem kosmetisch akzeptablen Lösungsmittel besteht.

**23.** Zusammensetzung nach Anspruch 22, **dadurch gekennzeichnet, dass** das Lösungsmittel unter den niederen Alkoholen mit 1 bis 4 Kohlenstoffatomen, wie Ethanol, Isopropanol, tert-Butanol, n-Butanol; Alkylenglycolen, wie Propylenglycol oder Hexylenglycol, Glycerol ausgewählt ist.

**24.** Zusammensetzung nach einem der Ansprüche 1 bis 23, **dadurch gekennzeichnet, dass** sie ferner einen oder mehrere Zusatzstoffe enthält, die unter den anionischen oder nichtionischen oder amphoteren Polymeren, Proteinen, Proteinhydrolysaten, Ceramiden, Pseudoceramiden, pflanzlichen Ölen, Fettsäuren, Hydroxysäuren, Vitaminen, Provitaminen wie Panthenol, Siliconen, die flüchtig oder nicht flüchtig, in dem Medium löslich oder unlöslich und von den sterisch gehinderten aminierten Siliconen verschieden sind, UV-Filtern, Hydratisierungsmitteln, Antischuppenmitteln, Wirkstoffen gegen Seborrhoe, Wirkstoffen gegen Haarausfall, Radikalfängern für freie Radikale, Trübungsmitteln und deren Gemischen ausgewählt sind.

**25.** Verwendung einer Zusammensetzung nach einem der vorhergehenden Ansprüche in der Kosmetik für die Reinigung und/oder für die Pflege und/oder zum Konditionieren und/oder zum Frisieren der Haare.

**26.** Verwendung einer Zusammensetzung nach einem der Ansprüche 1 bis 24 für die Reinigung und/oder zum Abschminken von Keratinsubstanzen.

**27.** Verfahren für die Reinigung und Pflege von Keratinsubstanzen, wie Haaren, das darin besteht, eine wirksame Menge einer Zusammensetzung nach einem der Ansprüche 1 bis 24 auf die feuchten Keratinsubstanzen aufzutragen und anschließend nach einer gegebenenfalls abgewarteten Einwirkzeit mit Wasser zu spülen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- GB 1513672 A
- US 2003130760 A
- EP 0739625 A
- EP 0974335 A
- WO 9746211 A
- US 2528378 A
- US 2781354 A
- EP 0337354 A
- FR 2270846 A
- FR 2383660 A
- FR 2598611 A
- FR 2470596 A
- FR 2519863 A

### Littérature non-brevet citée dans la description

- CTFA. 1993
- **M.R. PORTER.** Handbook of Surfactants. 1991, 116-178